(19) Europäisches Patentamt / European Patent Office / Office européen des brevets

(11) **EP 2 150 280 B1**

(12) **EUROPÄISCHE PATENTSCHRIFT**

(45) Veröffentlichungstag und Bekanntmachung des Hinweises auf die Patenterteilung:
**13.10.2010 Patentblatt 2010/41**

(21) Anmeldenummer: 08734890.0

(22) Anmeldetag: **31.03.2008**

(51) Int Cl.:
*A61L 2/12* *(2006.01)*    *A47L 15/48* *(2006.01)*
*H05B 6/80* *(2006.01)*

(86) Internationale Anmeldenummer:
**PCT/EP2008/002527**

(87) Internationale Veröffentlichungsnummer:
**WO 2008/145217 (04.12.2008 Gazette 2008/49)**

(54) **REINIGUNGSGERÄT MIT KEIMREDUKTION DURCH MIKROWELLEN**

CLEANING APPLIANCE WITH SYSTEM FOR GERM REDUCTION USING MICROWAVES

APPAREIL DE NETTOYAGE À RÉDUCTION DES GERMES PAR MICRO-ONDES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **30.05.2007 DE 102007025263**

(43) Veröffentlichungstag der Anmeldung:
**10.02.2010 Patentblatt 2010/06**

(73) Patentinhaber: **MEIKO Maschinenbau GmbH & Co. KG**
**77652 Offenburg (DE)**

(72) Erfinder:
• **GAUS, Bruno**
**77654 Offenburg (DE)**
• **NÄGER, Thomas**
**77652 Offenburg (DE)**
• **PEUKERT, Thomas**
**77815 Bühl (DE)**
• **SCHERINGER, Stefan**
**77654 Offenburg (DE)**
• **WIEGAND, Ingo**
**77830 Bühlertal (DE)**

(74) Vertreter: **Hörschler, Wolfram Johannes et al**
**Isenbruck Bösl Hörschler Wichmann LLP**
**Eastsite One**
**Seckenheimer Landstrasse 4**
**68163 Mannheim (DE)**

(56) Entgegenhaltungen:
WO-A-2006/079801    DE-A1- 3 701 366
DE-A1- 3 843 039    DE-A1- 3 843 040
DE-A1- 4 109 829    FR-A- 2 813 779

EP 2 150 280 B1

**Beschreibung**

Die Erfindung betrifft ein Reinigungsgerät zur Reinigung von Reinigungsgut, welches mindestens eine Mikrowellendesinfektionsvorrichtung aufweist. Derartige Reinigungsgeräte werden beispielsweise im gewerblichen Bereich oder im Haushaltsbereich in Form von Geschirrspülmaschinen eingesetzt, oder im Krankenhaus- oder Pflegebereich in Form von Reinigungs- und Desinfektionsautomaten beziehungsweise Steckbeckenspülern. Weiterhin betrifft die Erfindung einen Dampferzeuger zum Einsatz in einem Reinigungsgerät sowie ein Verfahren zur Reinigung von Reinigungsgut in einem Reinigungsgerät.

Stand der Technik

Aus dem Stand der Technik sind Reinigungsgeräte zur Reinigung von Reinigungsgut oder Spülgut in zahlreichen verschiedenen Variationen bekannt. Die Ausgestaltung dieser Reinigungsgeräte hängt von verschiedenen Randbedingungen, wie der Art des zu reinigenden Spülgutes, den Verschmutzungen, dem Durchsatz oder ähnlichen Bedingungen ab.

Die nachfolgend beschriebene Erfindung ist auf zahlreiche Reinigungsgeräte zur Reinigung der verschiedensten Arten von Reinigungsgut anwendbar. Ein erster besonderer Schwerpunkt liegt im Bereich der Geschirrspülmaschinen. Derartige Geschirrspülmaschinen sind als Haushaltsgeräte sowie als gewerbliche Geschirrspülmaschinen für den Einsatz beispielsweise in Betriebskantinen, Krankenhäusern, Schulen, oder ähnlichen Einrichtungen mit Großküchen bekannt. Gewerbliche Geschirrspülmaschinen werden unterteilt in Durchlaufgeschirrspülmaschinen, bei welchen das zu spülende Reinigungsgut (z.B. Tassen, Teller, Besteck, Gläser, Tabletts oder ähnliches Spülgut) nacheinander mehrere Reinigungszonen durchläuft. Beispielsweise kann eine derartige Durchlaufgeschirrspülmaschine eine Vorabräumzone, eine Waschzone eine Klarspülzone und eine Trocknungszone aufweisen. Auch Geschirrspülmaschinen mit weniger Reinigungszonen sind bekannt. Durchlaufgeschirrspülmaschinen sind im Stand der Technik beispielsweise als Bandtransport- oder Korbtransportmaschinen ausgestaltet.

Einkammergeschirrspülmaschinen hingegen weisen üblicherweise eine einzige Spülkammer als Reinigungszone auf, in welcher wiederum Reinigungsgut, beispielsweise in Form von Tassen, Tellern, Gläsern, Besteck, Geschirr oder Tabletts gereinigt werden kann. Üblicherweise werden Einkammergeschirrspülmaschinen für den Haushaltsbereich im Wasserwechsel betrieben, d.h. ein und derselbe Tank wird üblicherweise für ein Spülprogramm und einen Nachspülschritt verwendet, wobei zwischen diesen Schritten ein Austausch des Wassers beziehungsweise der Reinigungsflüssigkeit im Tank erfolgt. Um den Durchsatz zu erhöhen, werden für den gewerblichen Einsatz Einkammergeschirrspülmaschinen eingesetzt, welche neben einem Waschtank einen Nachspültank aufweisen, beispielsweise einen Boiler. In diesem Nachspültank kann Nachspülflüssigkeit (beispielsweise Wasser mit einem Klarspülerzusatz) auf eine Nachspültemperatur aufgeheizt werden, während der eigentliche Waschvorgang in der Spülkammer abläuft.

Ein weiterer Schwerpunkt der vorliegenden Anmeldung liegt auf Reinigungsgeräten, welche für die Reinigung von medizinischem Gerät und/oder Pflegegerät geeignet sind. Insbesondere kann es sich dabei um Reinigungsgut handeln, bei welchem große Mengen an Flüssigkeitsabfällen und/oder Festabfällen anfallen, welche entsorgt werden müssen. Als Beispiel seien dabei Nachtgeschirre, Steckbecken, Bettpfannen, Urinflaschen oder ähnliche Gefäße genannt, bei welchen leicht Flüssigkeitsmengen von mehr als einigen 10 ml bis hin in den Literbereich anfallen können. Derartige Reinigungsgeräte werden im Folgenden ohne Beschränkung des Reinigungsgutes auf ein bestimmtes Pflegegerät als "Steckbeckenspüler" bezeichnet.

Sowohl bei den Geschirrspülmaschinen als auch besonders bei den Steckbeckenspülern, sowie auch bei anderen Arten von Reinigungsgeräten kann in vielen Fällen eine Hygienewirkung der Reinigung eine besondere Rolle spielen. So ist im Fall der Geschirrspülmaschinen sicherzustellen, dass sämtliche Arten von Reinigungs- oder Spülgut, welche unmittelbar oder mittelbar mit Speisen oder Getränken in Berührung kommen, anhaftende Verschmutzungen sicher entfernt werden. Eine darüber hinaus gehende Problematik ergibt sich bei den Steckbeckenspülern, bei welchen anhaftende Keime (Viren oder Bakterien) ein erhöhtes Infektionsrisiko bergen können. Insofern ist in vielen Fällen eine Hygienisierung des Reinigungsgutes erforderlich. Diese Hygienisierung kann von einer einfachen Keimverminderung über eine Desinfektion (beispielsweise eine Desinfektion mit chemischen Desinfektionsmitteln und/oder eine thermische Desinfektion mit Wasserdampf oder einer anderen Art der Wärmebeaufschlagung) bis hin zu einer vollständigen Sterilisation des Reinigungsgutes reichen. Im Folgenden wird allgemein und ohne Beschränkung des Schutzumfangs der Erfindung überwiegend der Begriff "Desinfektion" verwendet, wobei dieser Begriff allgemein eine einfache Keimreduktion beinhalten soll, jedoch auch bis hin zu nahezu vollständiger Keimvernichtung und Sterilisation von Reinigungsgut reichen soll.

Zur Beurteilung der erreichten Hygienisierung des Reinigungsgutes in Reinigungsgeräten werden üblicherweise Testverfahren herangezogen, welche auf der Keimreduktion bestimmter Testkeime an der Reinigungsgutoberfläche basieren. Hierfür existieren auch Normen für verschiedene Arten von Reinigungsgeräten, auf welche die vorliegende Erfindung anwendbar ist.

So existiert in Deutschland beispielsweise die DIN 10511-12 C.3 für Geschirrspülmaschinen. In dieser Norm wird ein Verfahren beschrieben, bei dem mit definiert angeschmutzten Gläsern die Keimreduktion nach Programmablauf über so genannte Abklatschuntersuchungen ermittelt wird. Hierbei muss im kürzesten beziehungsweise ungünstigen Spülprogramm eine Mindest-Keimreduktion erreicht werden. Als Testkeim beziehungsweise Organismus wird bei diesem Test E. faecium ATCC 6057 verwendet. Aus den Ergebnissen dieser Untersuchung ergeben sich dann Mindestanforderungen hinsichtlich Temperatur, Spüldauer, Reinigerkonzentration und mechanischer Spülleistungen, mit der dann dieses Reinigungsgerät werksseitig voreingestellt werden muss, um im Betrieb beim Kunden die geforderte Keimreduktion zu erreichen.

Aus den USA ist ein Verfahren bekannt, welches überwiegend für Geschirrspülmaschinen eingesetzt wird und welches im NSF-Standard 3 beschrieben wird. Basis dieses Verfahrens ist die aus Versuchen ermittelte Keimreduktion von Tuberkulosebakterien durch die Einwirkung einer Temperatur über die Zeit. Das Einwirken der Temperatur über die Zeit wird als "Wärmeäquivalent" (HUE) bezeichnet. Wie viele Wärmeäquivalente pro Sekunde bei welcher Temperatur erzielt werden, ist in einer Tabelle, die diesem Verfahren zugrunde gelegt wird, niedergeschrieben. Anhand von Versuchen wurden für Geschirrspülmaschinen eine Mindesttanktemperatur und eine Mindestnachspültemperatur definiert, welche die Geschirrspülmaschine erreichen muss, um die geforderte Keimreduktion zu erreichen. Für den Hersteller von Geschirrspülmaschinen bedeutet dies, dass diese Temperaturen werksseitig fest in der Spülmaschinensteuerung der Geschirrspülmaschine voreingestellt werden müssen.

Bei der Verfahrensprüfung einer Geschirrspülmaschine nach diesem NSF-3-Verfahren wird ein Temperatursensor auf einem Teller angebracht. Anschließend wird dieser Teller in einer vorgegebenen Position im Geschirrkorb der Geschirrspülmaschine platziert und die Temperatur während des Programmablaufs aufgezeichnet. Aus dem Temperaturverlauf während des gesamten Spülprogramms werden dann anhand der oben erwähnten Tabelle die auf dem Teller über den gesamten Programmablauf einwirkenden Wärmeäquivalente ermittelt. Diese Prüfung ist für drei verschiedene Tellerpositionen innerhalb des Geschirrkorbs vorgeschrieben. Zur Erfüllung der geforderten Keimreduzierung müssen nach der Vorschrift NSF-3 mindestens 3600 Wärmeäquivalente in jeder Tellerposition innerhalb des Geschirrkorbs erreicht werden. Vorteilhaft an diesem Verfahren ist, dass dieses Verfahren mit relativ wenig Aufwand vor Ort beim Kunden durchgeführt werden kann, um die einwandfreie Funktion der Spülmaschine zu überprüfen.

Im europäischen Bereich gilt für Reinigungs-/Desinfektionsgeräte, wie beispielsweise Steckbeckenspüler, die EN ISO 15883-1, welche zur Beurteilung der Hygienewirkung ebenfalls den Zusammenhang zwischen der Keimreduzierung und der Temperatur über die Zeit heranzieht. Dieser Zusammenhang wird als $A_0$-Wert bezeichnet und ist ebenfalls in einer Tabelle niedergeschrieben beziehungsweise errechnet sich gemäß einer

mathematischen Beziehung. Der $A_0$-Wert ist definiert als das Zeitäquivalent in Sekunden bei einer Temperatur von 80 °C, bei dem eine gegebene Desinfektionswirkung ausgeübt wird, und entspricht sinngemäß den Wärmeäquivalenten (HUE) gemäß des NSF-Standards 3. Auch hierbei ist ein mindestens zu erreichender $A_0$-Wert an jeder Stelle in der Spülkammer des Reinigungs- und Desinfektionsgerätes und des Reinigungsguts sicherzustellen. Dies bezieht sich demnach auch auf die ungünstigste Stelle bei dem ungünstigsten Programm. Dieses Verfahren wird derzeit nicht zur Beurteilung der Hygienewirkung gewerblicher Spülmaschinen im europäischen Raum herangezogen.

Allen oben skizzierten Verfahren ist gemeinsam, dass stets das ungünstigste Spülprogramm beziehungsweise die ungünstigsten Bedingungen zur Beurteilung der Keimreduktion herangezogen werden. Nachteilig an diesem Vorgehen ist jedoch der Umstand, dass sich durch die starren Vorgaben für die Spülprogramme, die sich hinsichtlich Temperatur oder weiterer Programmparameter von diesen Vorgaben unterscheiden, unter Umständen unnötig lange Programmlaufzeiten ergeben, was höchst unerwünscht ist. So könnte zum Beispiel durch eine Erhöhung der Wasch- und/oder Nachspültemperatur die Programmlaufzeit bei gleichbleibender Keimreduktion verkürzt werden.

Weiterhin ist an den bekannten Verfahren zur Keimreduktion und den entsprechenden Normen als nachteilig zu verzeichnen, dass diese in der Regel mit einem enormen Energieverbrauch verbunden sind. Insbesondere im Bereich der gewerblichen Geschirrspülmaschinen und im Bereich der Reinigung von Krankenhaus- und Pflegebedarf macht sich dieser Nachteil jedoch gravierend bemerkbar. So kann eine thermische Desinfektion einen hohen Prozentsatz des gesamten Energieverbrauchs des Reinigungsgerätes ausmachen. Alternativ oder zusätzlich werden, je nach Kontaminationsgraden, auch chemische Desinfektions- oder Keimreduktionsverfahren eingesetzt, welche jedoch aufgrund der Aggressivität der verwendeten Chemikalien mit einer erheblichen Umweltbelastung und ebenfalls mit stark erhöhten Kosten für den Reinigungsprozess verbunden sein können.

Aufgabe der Erfindung

Aufgabe der Erfindung ist es daher, ein Reinigungsgerät bereitzustellen, welches die oben dargestellten Nachteile bekannter Reinigungsgeräte zumindest weitgehend vermeidet. Insbesondere soll das Reinigungsgerät eine energiesparende, kostengünstige und umweltfreundliche Keimreduktion des Reinigungsgutes ermöglichen.

Beschreibung der Erfindung

Diese Aufgabe wird durch die Erfindung mit den Merkmalen des unabhängigen Anspruchs gelöst. Vorteilhafte Weiterbildungen der Erfindung sind den Unteransprüchen gekennzeichnet. Der Wortlaut sämtlicher Ansprüche wird hiermit durch Bezugnahme zum Inhalt dieser Beschreibung gemacht.

Es wird erfindungsgemäß ein Reinigungsgerät zur Reinigung von Reinigungsgut vorgeschlagen, welches mindestens eine Reinigungszone zur Reinigung des Reinigungsgutes mit einer Reinigungsflüssigkeit aufweist. Beispielsweise kann das Reinigungsgerät einem bekannten Reinigungsgerät gemäß der obigen Beschreibung des Standes der Technik entsprechen und kann beispielsweise eine Durchlaufgeschirrspülmaschine, eine Einkammergeschirrspülmaschine (insbesondere für den gewerblichen Einsatz) und/oder einen Steckbeckenspüler umfassen.

Ein Grundgedanke der vorliegenden Erfindung besteht darin, die Problematik der bislang bekannten chemischen und/oder thermischen Keimreduktionen in üblichen Reinigungsgeräten dadurch zu umgehen oder zu vermindern, dass Mikrowellenstrahlung für die Hygienisierung eingesetzt wird. Unter Mikrowellenstrahlung wird dabei im Folgenden elektromagnetische Strahlung verstanden, welche in einem Frequenzbereich zwischen 0,3 und 300 GHz liegt. Diese Mikrowellenstrahlung kann direkt (das heißt durch direkte Bestrahlung des zu hygienisierenden Gutes) und/oder indirekt zur Hygienisierung des Reinigungsgutes eingesetzt werden. Unter einer "indirekten" Hygienisierung ist dabei eine Hygienisierung zu verstehen, bei welcher die Mikrowellen genutzt werden; um nicht unmittelbar Keime abzutöten, sondern um eine Erwärmung eines weiteren Gegenstandes zu bewirken, wobei diese Erwärmung wiederum die keimreduzierende Wirkung herbeiführt. Beispielsweise kann dieser dritte Gegenstand das zu reinigende Reinigungsgut selbst sein (zum Beispiel Geschirr, Tassen, Gläser, Besteck, Tabletts, Bettpfannen, Urinflaschen, Steckbecken, Nachtgeschirr oder ähnliches), ein Flüssigkeitsfilm, welcher auf diesem Reinigungsgut aufgenommen ist, Wasser, welches dann wiederum zur Keimreduktion auf das Reinigungsgut aufgebracht wird, Dampf oder ähnliches.

Es sei dabei darauf hingewiesen, dass, in Abweichung von üblichen Begriffen, die Begriffe "Hygienisierung", "Desinfektion", "Keimreduktion" und "Sterilisation" synonym verwendet werden, ohne hierdurch den Grad der Hygienisierung auszudrücken. Die Hygienisierung kann von einer einfachen Keimreduktion (beispielsweise einer Reduktion einer Kontamination mit einem oder mehreren Erregern oder Keimen, beispielsweise Bakterien oder Viren) um wenige Prozent (beispielsweise 10 %) bis hin zu einer nahezu vollständigen Keimvernichtung (Sterilisation) reichen. Bevorzugt ist es, wenn die Hygienisierung derart durchgeführt wird, dass mehr als 90 % der Keime abgetötet werden.

Aus verschiedenen anderen Bereichen ist der Einsatz von Mikrowellenstrahlung aus dem Stand der Technik bekannt. Beispielsweise werden Mikrowellenstrahlungen großtechnisch zur Trocknung von feuchten Gemäuern im Baubereich eingesetzt. Andere Einsatzgebiete sind beispielsweise die Lackherstellung und die Beschichtungstechnologie, in welchen Mikrowellenverfahren zur Trocknung der Beschichtungen eingesetzt werden (siehe zum Beispiel EP 1 327 844 A2). Der Grundgedanke der vorliegenden Erfindung besteht jedoch darin, die Mikrowellenstrahlung nicht, beziehungsweise nicht ausschließlich, zur Trocknung einzusetzen, sondern gezielt zur Keimreduktion in Reinigungsgeräten zu verwenden.

Dementsprechend wird vorgeschlagen, das Reinigungsgerät mit einer entsprechenden Mikrowellendesinfektionsvorrichtung auszustatten. Diese Mikrowellendesinfektionsvorrichtung (wobei auch mehrere derartiger Vorrichtungen vorgesehen sein können) soll eine Mikrowellenquelle zur Erzeugung der Mikrowellenstrahlung aufweisen. Beispielsweise kann diese Mikrowellenquelle übliche bekannte Arten von Mikrowellenerzeugern umfassen, wobei aufgrund der gezielt einstellbaren Eigenschaften der erzeugten Strahlung die Verwendung von Magnetrons bevorzugt ist. Alternativ oder zusätzlich sind jedoch auch andere Arten von Mikrowellenquellen einsetzbar.

Wie oben beschrieben, soll die Mikrowellendesinfektionsvorrichtung eingerichtet sein, um eine direkte oder indirekte Keimreduktion zu bewirken. Dies kann beispielsweise dadurch erfolgen, dass, wie oben angedeutet, eines oder mehrere der folgenden Funktionsprinzipien eingesetzt wird:

- Mittels der Mikrowellenstrahlung wird in einem Dampferzeuger der Mikrowellendesinfektionsvorrichtung Dampf erzeugt, welcher eingesetzt wird, um eine Keimreduktion durchzuführen (indirekte Hygienisierung).

- Die Mikrowellenstrahlung wird auf das Reinigungsgut ausgerichtet, wobei die Mikrowellenstrahlung eingerichtet ist, um einen Flüssigkeitsfilm der sich aus den vorangegangenen Reinigungsvorgängen noch auf der Oberfläche des Reinigungsguts befindet zu erwärmen. Diese Erwärmung erfolgt vorzugsweise bis zu einer derartigen Temperatur, dass Keime, die sich auf den Oberflächen des Reinigungsguts befinden, im Wesentlichen abgetötet werden. Die Temperatur an der Oberfläche des Reinigungsgutes kann beispielsweise durch einen oder mehrere Temperatursensoren überwacht werden. Diese Erwärmung eines Flüssigkeitsfilms an der Oberfläche des Reinigungsgutes ist wiederum eine indirekte Hygienisierung.

- Die Mikrowellenstrahlung wird auf das Reinigungsgut ausgerichtet, wobei die Mikrowellenstrahlung eingerichtet ist, um das Reinigungsgut zu erwärmen. Diese Erwärmung erfolgt vorzugsweise bis zu einer derartigen Temperatur, dass Keime im Wesentlichen abgetötet werden. Beispielsweise kann die Temperatur des Reinigungsgutes durch einen oder mehrere Temperatursensoren überwacht werden. Die Mikrowellenstrahlung wird von ihrem Frequenzbereich her vorzugsweise derart gewählt, dass diese zumindest teilweise von dem Reinigungsgut absorbiert wird. Dies ist vergleichsweise einfach möglich, da insbesondere im gewerblichen Bereich üblicherweise nur wenige, zumeist bekannte Arten von Reinigungsgut in der Reinigungsvorrichtung gereinigt werden (zum Beispiel Teller), wobei die Absorption der dort verwendeten Materialien bekannt sein kann. Diese Erwärmung des Reinigungsgutes ist wiederum eine indirekte Hygienisierung.

- Die Mikrowellenstrahlung wird auf das Reinigungsgut ausgerichtet, wobei die Mikrowellenstrahlung eingerichtet ist, um Keime auf und/oder in dem Reinigungsgut und/oder auch im gesamten Reinigungsgerät (zum Beispiel einer Kammer des Reinigungsgerätes) zu reduzieren. Bei dieser Hygienisierung handelt es sich um eine direkte Hygienisierung, wobei die Mikrowellenstrahlung von ihrer Frequenz vorzugsweise derart eingestellt ist, dass diese unmittelbar auf Absorptionseigenschaften der Keime eingestellt ist. Insbesondere sollen bekannte Bakterien und/oder Viren diese Mikrowellenstrahlung zumindest teilweise absorbieren. Bei dieser Hygienisierung handelt es sich um eine direkte Hygienisierung.

- Die Mikrowellendesinfektionsvorrichtung weist einen Dampferzeuger auf, wobei das Reinigungsgerät eingerichtet ist, um das Reinigungsgut mit von dem Dampferzeuger erzeugtem Dampf zu beaufschlagen. Im Unterschied zum oben genannten, ersten Hygienisierungsprinzip kann der Dampferzeuger jedoch eine "konventionelle" Dampferzeugung (zum Beispiel mittels einer Heizung) umfassen. Die Mikrowellendesinfektionsvorrichtung ist jedoch eingerichtet, um mittels der Mikrowellenstrahlung den Dampf zu überhitzen. Dieses Prinzip ermöglicht es, indem der Dampf die Mikrowellenstrahlung zumindest teilweise absorbiert, diesen Dampf innerhalb des Reinigungsgerätes zu überhitzen, ohne dass hierdurch erhöhte Drücke erforderlich wären. Auf diese Weise kann, beispielsweise auch bei Normaldruck oder lediglich leichtem Überdruck oder sogar einem Unterdruck, der Dampf in dem Reinigungsgerät auf über 100 °C erhitzt werden, wodurch die Keimreduktion stark verbessert wird.

- Die vorgeschlagene Mikrowellenhygienisierung weist gegenüber herkömmlichen Hygienisierungsverfahren zahlreiche entscheidende Vorteile auf. So kann beispielsweise das Reinigungsgut keimspezifisch beaufschlagt werden, wobei die Mikrowellenstrahlung beispielsweise unmittelbar auf einen abzutötenden Zielkeim oder Zielerreger oder eine Gruppe derartiger Erreger oder Keime eingestellt wird. Auf diese Weise ist die Effizienz der Keimreduktion erheblich erhöht.

- Weiterhin ist eine unspezifische gesamte Aufheizung der Reinigungskammer beziehungsweise der Reinigungszone nicht oder lediglich in wesentlich geringerem Umfang erforderlich, im Gegensatz zu bekannten thermischen Hygienisierungsverfahren. Die Mikrowellenstrahlung kann beispielsweise gezielt auf das Reinigungsgut eingestrahlt werden, so dass die Leistungs- oder Energiedichte gezielt lediglich im Bereich dieses Reinigungsgutes erhöht werden kann. Hierdurch lässt sich eine erhebliche Einsparung an Energie bewirken.

Auch bei der Dampferzeugung zur Keimreduktion lassen sich erhebliche Mengen an Energie einsparen. Dementsprechend wird, unabhängig von dem Reinigungsgerät, auch ein Dampferzeuger beansprucht, welcher auf dem Prinzip der Dampferzeugung durch eine Mikrowellenquelle basiert und welcher für den Einsatz in einem Reinigungsgerät geeignet ist. Im Gegensatz zu aus dem Stand der Technik bekannten Dampferzeugern, welche üblicherweise eine Heizung in einem Tank zur Dampferzeugung einsetzen, kommt dieser Mikrowellen-Dampferzeuger mit kleinsten zu verdampfenden Flüssigkeitsmengen aus. Während in thermischen Dampferzeugern beispielsweise eine Problematik darin besteht, dass eine Heizwendel vollständig bedeckt sein muss, wodurch eine Mindestwassermenge vorgegeben ist, lassen sich Dampferzeuger nach diesem neuen Prinzip bereits mit wenigen Millilitern Wasser beziehungsweise anderer Flüssigkeit betreiben, wodurch wiederum eine Effizienz der Dampferzeugung stark verbessert werden kann.

Nichtsdestotrotz lassen sich jedoch auch herkömmliche Hygienisierungsverfahren, beispielsweise thermische und/oder chemische Hygienisierungsverfahren, mit dem vorgeschlagenen Mikrowellen-Hygienisierungsverfahren kombinieren. Durch den Einsatz der Mikrowellen lässt sich jedoch der Aufwand, beispielsweise der Energieaufwand und/oder die Menge eines zugegebenen chemischen Desinfektionsmittels, erheblich vermindern, und die Dimensionierung beispielsweise von Heizquellen für die thermische Desinfektion lässt sich reduzieren.

Die Mikrowellendesinfektionsvorrichtung kann mindestens einen Hohlleiter zum Ausrichten der Mikrowellenstrahlung aufweisen. Der Hohlleiter kann insbesondere genutzt werden, um die Mikrowellen von der Mikrowellenquelle zu einem Gehäuse, beispielsweise einem Innenraum der Reinigungszone (zum Beispiel einem Innenraum einer Spülkammer) zu leiten. Insbesondere kann der mindestens eine Hohlleiter auch genutzt werden, um die Mikrowellen auf das Reinigungsgut auszurichten, beispielsweise indem dieser Hohlleiter eine entsprechend auf das Reinigungsgut ausgerichtete Öffnung aufweist. Eine Öffnung des Hohlleiters im Inneren der Reinigungszone kann eine verschließbare Öffnung aufweisen, beispielsweise eine Öffnungsklappe. Insbesondere kann diese Öffnungsklappe derart ausgestaltet sein, dass diese verhindert, dass Reinigungsflüssigkeit in den Hohlleiter und/oder in die Mikrowellenquelle eindringt. Beispielsweise kann die Öffnungsklappe eine federgelagerte Öffnungsklappe aufweisen, welche durch ein Gebläse der Mikrowellendesinfektionsvorrichtung automatisch geöffnet wird. Auch ein automatisch (zum Beispiel elektromechanisch) zu öffnendes Ventil kann an der Öffnung vorgesehen sein. Unter "verschließbar" ist jedoch auch eine andere Art von Abschirmung der Öffnung zu verstehen, beispielsweise eine für Mikrowellen zumindest teilweise transparente Abdeckung, welche jedoch Wasser vom Inneren des Hohlleiters abweist.

Die Mikrowellendesinfektionsvorrichtung kann weiterhin eingerichtet sein, um Warmluft auf das Reinigungsgerät aufzubringen. Beispielsweise kann der oben beschriebene Hohlleiter genutzt werden, um Warmluft zum Reinigungsgut zu leiten. Besonders bevorzugt ist es dabei, wenn die Mikrowellendesinfektionsvorrichtung ein Gebläse aufweist, welches zum Erzeugen der Warmluft genutzt wird. Zu diesem Erzeugen der Warmluft kann eine Mikrowellenquelle selbst genutzt werden, da diese in der Regel Abwärme produziert. Beispielsweise kann ein Kühlgebläse der Mikrowellendesinfektionsvorrichtung genutzt werden, welches zunächst Luft auf die Mikrowellenquelle leitet, wobei die Luft dort erwärmt wird, um anschließend diese Luft auf das Reinigungsgut aufzubringen. Auf diese Weise kann beispielsweise ein Trocknungseffekt von feuchtem Spülgut erzielt werden und/oder der Hygienisierungseffekt der Mikrowellenstrahlung kann durch Warmluft unterstützt werden, ohne dass hierfür ein separates Gebläse mit einer Vorrichtung zum Erwärmen der Luft erforderlich wäre. Auf diese Weise lässt sich erheblich Energie einsparen.

Die Mikrowellenquelle kann im Dauerstrichbetrieb oder im Impulsbetrieb betrieben werden. Dabei kann die Mikrowellendesinfektionsvorrichtung eingerichtet sein, um Mikrowellenstrahlung mit einer einzelnen Frequenz oder innerhalb eines einzelnen Frequenzbandes zu erzeugen. Alternativ oder zusätzlich kann die Mikrowellendesinfektionsvorrichtung auch eingerichtet sein, um Mikrowellenstrahlung mit mindestens zwei Frequenzen zu erzeugen. Beispielsweise kann Mikrowellenstrahlung erzeugt werden, welche besonders gut vom Reinigungsgut absorbiert wird, und Mikrowellenstrahlung, welche einen unmittelbaren Desinfektionseffekt auf Keime auf dem Reinigungsgut hat. Besonders bevorzugt ist es dabei, wenn die Mikrowellenstrahlung derart gewählt wird, dass diese nicht oder nur geringfügig von den Werkstoffen des Reinigungsgerätes absorbiert wird, beispielsweise von Kammerwänden einer Reinigungszone und/oder einer Spülkammer. Dies ist mit modernen Mikrowellenquellen, beispielsweise dem oben beschriebenen Magnetron, heute problemlos möglich. Zudem können Reinigungsgeräte auch entsprechend mit geeigneten Materialien ausgestaltet werden, welche beispielsweise ganz oder teilweise transparent (das heißt nicht oder nur geringfügig absorbierend) für die verwendete Mikrowellenstrahlung sind, sich also nicht wesentlich aufheizen.

Die Mikrowellendesinfektionsvorrichtung kann insbesondere derart eingerichtet sein, dass Mikrowellenstrahlung mit mindestens einer Frequenz emittiert wird, welche zumindest teilweise vom Reinigungsgut absorbiert wird. Alternativ oder zusätzlich kann Mikrowellenstrahlung erzeugt werden, welche von an dem Reinigungsgut anhaftender Reinigungsflüssigkeit absorbiert wird. Wiederum alternativ oder zusätzlich kann auch Mikrowellenstrahlung emittiert werden, welche zumindest teilweise von Keimen, insbesondere von an dem Reinigungsgut anhaftenden Keimen, absorbiert wird. Weiter alternativ oder zusätzlich kann Mikrowellenstrahlung verwendet werden, welche mindestens eine Frequenz aufweist, die zumindest teilweise von dem im Reinigungsgerät enthaltenen Wasserdampf absorbiert wird. Hier bietet sich beispielsweise das in üblichen Mikrowellengeräten verwendete Frequenzband bei ca. 2,5 GHz an.

Neben der Einstrahlung von einem oder mehreren fest vorgegebenen Frequenzen beziehungsweise Frequenzbändern kann die Mikrowellendesinfektionsvorrichtung auch derart eingerichtet sein, dass ein oder mehrere Frequenzbändern zeitlich variiert werden. Auf diese Weise können das beziehungsweise die Frequenzbänder nacheinander unterschiedliche Frequenzbereiche abdecken, indem die Mikrowellenstrahlung durchgestimmt wird. Auf diese Weise lässt sich kurzfristig eine höhere spektrale Energiedichte erzeugen, welche dann jedoch über einen bestimmten Bereich durchgestimmt wird. Hierdurch lässt sich die Effizienz der Desinfektion zusätzlich erhöhen.

Wie oben beschrieben, ist es bevorzugt, wenn das Reinigungsgerät mindestens einen Temperatursensor aufweist. Insbesondere kann dieser mindestens eine Temperatursensor eingesetzt werden, um eine Temperatur des Reinigungsgutes zu erfassen. Alternativ oder zusätzlich kann dieser mindestens eine Temperatursensor jedoch auch beispielsweise eine Umgebungstemperatur (zum Beispiel eine Dampftemperatur) in der mindestens einen Reinigungszone und/oder in einer separaten Desinfektionszone erfassen. Beispielsweise lassen sich als Sensoren Infrarotsensoren einsetzen. Alternativ oder zusätzlich können jedoch auch andere Arten von Temperatursensoren eingesetzt werden, beispielsweise resistive Temperatursensoren.

Besonders vorteilhaft ist eine Ausgestaltung des Reinigungsgerätes, in welcher dieses eingerichtet ist, um einem Benutzer eine Eingabe mindestens eines Zielkeims und/oder einer Gruppe von Zielkeimen zu ermöglichen. Diese Eingabe kann beispielsweise manuell erfolgen, zum Beispiel über eine Tastatur, einen Bildschirm (beispielsweise durch Auswahl aus einer Liste) oder andere manuelle Eingabegeräte. Alternativ kann die Eingabe jedoch auch durch einen Datenaustausch erfolgen, beispielsweise über eine Schnittstelle, zum Beispiel im Rahmen einer Einbindung des Reinigungsgerätes in ein Netzwerk in einem Krankenhaus und/oder Pflegeheim. Auf diese Weise können beispielsweise sämtliche Reinigungsgeräte oder einige Reinigungsgeräte innerhalb eines Krankenhauses oder Pflegeheimes gleichzeitig oder mit geringem Zeitversatz auf bekannt gewordene Erreger eingestellt werden. Beispielsweise kann ein Arzt oder ein Pfleger von einem zentralen Terminal aus mehrere Reinigungsgeräte darüber informieren, dass verstärkt mit dem Auftreten eines bestimmten Zielkeimes oder Zielerregers beziehungsweise einer Gruppe derartiger Zielkeime oder Zielerreger zu rechnen ist. Diese Informationen lassen sich beispielsweise aus Krankendatenbanken erhalten oder aus öffentlichen Informationen, beispielsweise im Rahmen eines Seucheninformationsnetzwerkes. Bei Auftreten bestimmter Seuchen oder Infektionen beziehungsweise einer erhöhten Wahrscheinlichkeit des Auftretens derartiger Seuchen oder Infektionen können dann gezielt die Reinigungsgeräte zentral eingestellt werden, um eine besonders hohe Hygienisierungseffizienz bis hin zu einer Desinfektion oder Sterilisation des Reinigungsgerätes speziell für den ausgewählten Zielkeim beziehungsweise die ausgewählten Zielkeime zu erzielen.

Diese Information über den Zielkeim beziehungsweise die Gruppe von Zielkeimen ermöglicht es, entsprechend der Auswahl des Zielkeims beziehungsweise der Zielkeime die Mikrowellendesinfektionsvorrichtung anzusteuern. Beispielsweise kann gezielt eine Zielkeim-spezifische Frequenz oder ein Zielkeim-spezifisches Frequenzband eingestellt werden. Alternativ oder zusätzlich kann auch eine Zielkeim-spezifische Einwirkzeit einer Desinfektion gewählt werden, beispielsweise einer der oben beschriebenen direkten oder indirekten Desinfektionsverfahren. Beispielsweise kann es sich um die Einwirkzeit einer unmittelbaren Mikrowellen-Desinfektion, das heißt einer unmittelbaren Keimabtötung durch Mikrowellen handeln, oder, alternativ oder zusätzlich, um eine Einwirkzeit einer Dampfdesinfektion, wobei der Dampf durch Mikrowellen erzeugt wird und/oder durch Mikrowellen überhitzt wird. Werden die Mikrowellen eingesetzt, um eine thermische Desinfektion (zum Beispiel durch Dampf) zu bewirken, so lassen sich wiederum beispielsweise die oben beschriebenen Hygienisierungsnormen verwenden, um eine zuverlässige Keimabtötung einschätzen zu können. Alternativ oder zusätzlich können jedoch auch Geräte-spezifische Erfahrungswerte gesammelt werden, welche beispielsweise in einer Liste hinterlegt werden können, um dann, entsprechend dem eingegebenen Zielkeim beziehungsweise den Zielkeimen, diese Erfahrungswerte für die Hygienisierung zu verwenden. Hierbei lassen sich rein empirisch gewonnene Erfahrungswerte einsetzen, oder es lassen sich auch analytisch oder semiempirisch gewonnene Informationen verwenden. Derartige empirische Erfahrungswerte sind beispielsweise durch entsprechende Abklatschuntersuchungen von Testkeimen leicht experimentell erhältlich, über welche beispielsweise die Auswirkung einer Mikrowellendesinfektion auf eine Zielkeim-Population leicht erfasst werden kann.

Weiterhin lässt sich, alternativ oder zusätzlich, auch eine Zielkeim-spezifische Wirkungsdosis einsetzen, wobei wiederum, beispielsweise entsprechend dem vorangehend beschriebenen Verfahren, Erfahrungswerte oder semiempirische oder analytische Werte eingesetzt werden können. Unter einer Wirkungsdosis soll dabei ein zeitliches Integral über eine Intensität der Mikrowellenstrahlung verstanden werden, welche auf dem Reinigungsgut auftrifft, jeweils multipliziert mit einem Wirkungsfaktor, der abhängig ist von der Frequenz beziehungsweise dem Frequenzband der verwendeten Mikrowellenstrahlung und der Art des Zielkeims:

$$D = \int I(f) \cdot R(f, K) \cdot dt \qquad (1),$$

wobei I(f) die Intensität (auftreffende Strahlungsleistung pro Fläche des Reinigungsgutes) bezeichnet, R(f, K) einen Wirkungsfaktor, welcher von der verwendeten Frequenz beziehungsweise dem Frequenzband f abhängt sowie von dem Zielerreger beziehungsweise den Zielerregern K. Integriert wird dabei über die gesamte Einwirkzeit der Mikrowellenstrahlung.

Diese Wirkungsdosis beziehungsweise die einzelnen Komponenten dieser Wirkungsdosis lassen sich beispielsweise wiederum empirisch durch Abklatschuntersuchungen entsprechend bestrahlter, mit bekannten Testkeimen kontaminierter Proben ermitteln und beispielsweise für verschiedene Testkeime tabellieren. Auf diese Weise kann beispielsweise die keimabtötende Wirkung, welche in den Wirkungsfaktor R eingeht, bestimmt werden, und es kann jeweils eine Beaufschlagung des Spülgutes mit einer entsprechenden Wirkungsdosis an Mikrowellenstrahlung erfolgen, entsprechend dem vorgegebenen Zielkeim beziehungsweise den vorgegebenen Zielkeimen.

Das Reinigungsgerät kann beispielsweise eine Steuerung umfassen, welche eingerichtet ist, um einen Programmablauf zu steuern. Beispielsweise kann dann in mindestens einem Programmschritt des Programmablaufs ein Keimreduktionsschritt durchgeführt werden, in welchem die Mikrowellendesinfektionsvorrichtung entsprechend angesteuert wird.

Wie oben beschrieben, kann das Reinigungsgerät insbesondere eine Durchlaufgeschirrspülmaschine umfassen beziehungsweise eine derartige Durchlaufgeschirrspülmaschine sein. Auch die Ausgestaltung beziehungsweise Einbindung einer Einkammergeschirrspülmaschine, insbesondere für den gewerblichen Bereich (das heißt vorzugsweise ohne Wasserwechsel, das heißt mit separatem Nachspültank), ist möglich. Weiterhin ist, alternativ oder zusätzlich, die Einbindung eines Steckbeckenspülers denkbar, welcher vorzugsweise für die Reinigung von medizinischem Gerät und/oder Pflegegerät mit größerem Flüssigkeits- oder Feststoffabfall ausgestaltet ist. Insbesondere kann dieser Steckbeckenspüler eine Spülkammer umfassen, einen Ablauf mit einem Siphonbogen und vorzugsweise eine von der Spülkammer unter Umgehung des Siphonbogens in den Ablauf mündende Abluftleitung. Durch diese Abluftleitung kann feuchte Luft aus der Spülkammer in den Ablauf gedrückt werden, beispielsweise durch ein separates Zuluftgebläse, welches zwangsweise Frischluft in die Spülkammer einbringt und feuchte Luft in den Ablauf verdrängt. Alternativ oder zusätzlich kann jedoch für diese Verdrängung, welche beispielsweise zur Kühlung des Reinigungsgutes verwendet werden kann, auch ein Gebläse der Mikrowellendesinfektionsvorrichtung genutzt werden. Dieses Gebläse soll eingerichtet sein, um feuchte Luft aus der Spülkammer in den Ablauf zu verdrängen. In beiden beschriebenen Fällen kann die Abluftleitung beispielsweise ein selbstsperrendes Ventil, insbesondere ein Rückschlagventil, aufweisen, welches beispielsweise genutzt werden kann, um eine Rückverkeimung des Reinigungsgutes in der Spülkammer durch aus dem Ablauf eindringende Luft zu verhindern.

Im Falle der Ausgestaltung des Reinigungsgerätes als Steckbeckenspüler, jedoch auch bei anderen Arten von Reinigungsgeräten mit einem Ablauf, ist es besonders bevorzugt, wenn die Mikrowellendesinfektionsvorrichtung eingerichtet ist, um in dem Ablauf aufgenommene Flüssigkeit zu erwärmen. Beispielsweise kann auf diese Weise Flüssigkeit im Ablauf desinfiziert werden. Alternativ oder zusätzlich kann diese Erwärmung der Flüssigkeit im Ablauf jedoch auch so weit gehen, dass der Ablauf selbst als Dampferzeuger genutzt wird. Beispielsweise kann aus der im Ablauf aufgenommenen Flüssigkeit Dampf erzeugt werden, um mittels dieses Dampfes Reinigungsgut in der Spülkammer zumindest teilweise zu hygienisieren.

Ausführungsbeispiele

Weitere Einzelheiten und Merkmale der Erfindung ergeben sich aus den nachfolgenden Beschreibungen von bevorzugten Ausführungsbeispielen in Verbindung mit den Unteransprüchen. Hierbei können die jeweiligen Merkmale für sich allein oder zu mehreren in Kombination miteinander verwirklicht sein. Die Erfindung ist nicht auf die Ausführungsbeispiele beschränkt.

Die Ausführungsbeispiele sind in den Figuren schematisch dargestellt. Gleiche Bezugsziffern in den einzelnen Figuren bezeichnen dabei gleiche oder funk- tionsgleiche beziehungsweise hinsichtlich ihrer Funktio- nen einander entsprechende Elemente.

Im Einzelnen zeigt:

Figur 1    ein Ausführungsbeispiel einer erfindungsgemäßen Durchlaufgeschirrspül- maschine;

Figur 2    ein Ausführungsbeispiel einer erfindungsgemäßen Einkammergeschirr- spülmaschine;

Figur 3    ein Ausführungsbeispiel eines erfindungsgemäßen Steckbeckenspülers; und

Figur 4    ein Ausführungsbeispiel eines erfindungsgemäßen Dampferzeugers.

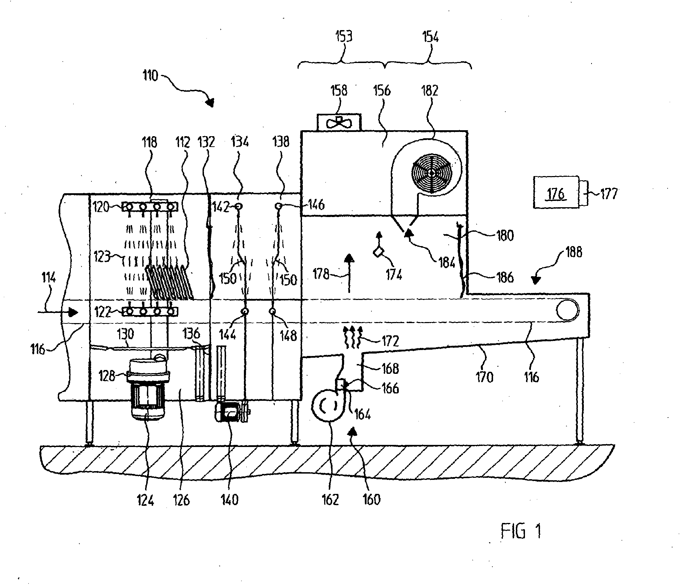

In Figur 1 ist ein erstes Ausführungsbeispiel ei- nes Reinigungsgerätes in schematischer Schnittdarstel- lung von der Seite gezeigt. In diesem Fall ist das Reinigungsgerät als eine Durchlaufgeschirrspülmaschine 110 ausgestattet. In dieser Durchlaufgeschirrspülmaschine 110 wird Reinigungsgut 112 in einer Durchlaufrichtung 114 bzw. Transportrichtung durch Behandlungszonen bzw. Reinigungszonen der Durchlaufgeschirrspülmaschine 110 transportiert. Eine Fördereinrichtung 116, die in der Darstellung gemäß Figur 1 als endloses Transportband ausgebildet ist, transportiert das zu reinigende Gut 112 durch die verschiedenen Reinigungszonen der Durchlaufgeschirrspülmaschine 110.

In Transportrichtung 114 des zu reinigenden Gutes 112 gesehen, passiert dieses zunächst eine Spülzone 118. Innerhalb der Spülzone 118 befindet sich ein erstes Spülsystem 120 sowie ein zweites Spülsystem 122. Aus diesem tritt in Strahlenform Reinigungsfluid 123 aus. Das erste Spülsystems 120 und das zweite Spülsystems 122 sind über eine erste Pumpe 124 mit Reinigungsfluid beaufschlagt. Die erste Pumpe 124 ist innerhalb eines Spülzonentanks 126, der der Spülzone 118 zugeordnet ist, untergebracht. Im oberen Bereich der ersten Pumpe 124 befindet sich ein Pumpengehäuse 128. Der Spülzonentank 126 ist mittels eines Tankabdecksiebes 130 abgedeckt. Der der Spülzone 118 zugeordnete Spülzonentank 126 enthält einen beheizten oder unbeheizten Wasservorrat.

Die Spülzone 118 ist mittels eines Trennvorhangs 132 von der sich - in Transportrichtung 114 des zu reinigenden Gutes 112 gesehen - an diese anschließenden Pumpenklarspülzone 134 getrennt. Der Spülzonentank 126 ist über eine Trennwand 136 von dem Tank getrennt, der sich unterhalb der Pumpenklarspülzone 134 bzw. einer sich an die Pumpenklarspülzone 134 anschließenden Frischwasserklarspülzone 138 befindet.

In der Darstellung gemäß Figur 1 läuft das die Spülzone 118 verlassende zu reinigende Gut 112 nach Passage des Trennvorhangs 132 in die Pumpenklarspülzone 134 ein. Die Pumpenklarspülzone 134 wird über eine zweite Pumpe 140 gespeist. Das in der Pumpenklarspülzone 134 aus einem ersten Sprührohr 142 und einem zweiten Sprührohr 144 austretende Reinigungsfluid 123 benetzt das zu reinigende Gut 112 von der Oberseite und der Unterseite her. Die in der Pumpenklarspülzone 134 angeordneten Sprührohre 142, 144 sind an einem gebogenen Rohr aufgenommen, so dass ein Versatz des ersten Sprührohres 142 im Vergleich zum zweiten Sprührohr 144 der Pumpenklarspülzone 134 erreicht wird.

Gleiches gilt für die Frischwasserklarspülzone 138, die der Pumpenklarspülzone 134 nachgeschaltet sein kann. Die Frischwasserklarspülzone 138 umfasst ein oberes Sprührohr 146 und ein unteres Sprührohr 148. Die beiden Sprührohre 146, 148 sind entsprechend des Sprührohrverlaufes 150 in Transportrichtung 114 des zu reinigenden Gutes 112 gesehen ebenfalls zueinander versetzt angeordnet. Das aus dem oberen Sprührohr 146 und dem unterem Sprührohr 148 austretende Frischwasservolumen benetzt das reinigende Gut 112 von dessen Oberseite und dessen Unterseite her mit einem Frischwasserstrahl 152.

Der Frischwasserklarspülzone 138 ist eine Desinfektionszone 153 und eine Trocknungszone 154 nachgeordnet. Diese Zonen 153, 154 können beispielsweise von der Frischwasserklarspülzone 138 durch einen weiteren Vorhang (in Figur 1 nicht dargestellt) getrennt sein.

Bei dem in Figur 1 dargestellten, den Umfang der Erfindung nicht beschränkenden Ausführungsbeispiel ist in der Desinfektionszone 153 eine Wärmerückgewinnungseinrichtung 156 angeordnet. Diese Wärmerückgewinnungseinrichtung 156 umfasst ein Abluftgebläse 158, mittels dessen Abluft aus der Durchlaufgeschirrspülmaschine 110 abgezogen wird. Beispielsweise kann die Wärmerückgewinnungseinrichtung 156 einen Wärmetauscher umfassen, über welchen Wärmetauscherflüssigkeit aufgewärmt wird, um anschließend beispielsweise in dem Spülzonentank 126 eingesetzt zu werden. Auf diese Weise wird die abgeführte Wärme zumindest teilweise wieder verwendet, so dass der Energiebedarf für eine Tankheizung des Spülzonentanks 126 gesenkt werden kann. Weiterhin kann die Wärmerückgewinnungseinrichtung 156 auch eine Kondensatniederschlagseinrichtung (nicht dargestellt) umfassen, um feuchte Luft zumindest teilweise zu kondensieren.

Weiterhin ist in der Desinfektionszone 153 eine Mikrowellendesinfektionsvorrichtung 160 vorgesehen. In dem in Figur 1 dargestellten Ausführungsbeispiel ist dabei lediglich eine derartige Mikrowellendesinfektionsvorrichtung 160 dargestellt, wobei naturgemäß auch mehrere derartiger Einrichtungen vorgesehen sein können, beispielsweise um das zu reinigende Gut 112 von mehreren Seiten mit Mikrowellen zu beaufschlagen. Beispielsweise kann eine weitere Mikrowellendesinfektionsvorrichtung 160 im Deckenbereich der Desinfektionszone angeordnet sein, beispielsweise um gezielt Telleroberflächen mit Mikrowellenstrahlung zu beaufschlagen.

Die Mikrowellendesinfektionsvorrichtung 160 umfasst in diesem Ausführungsbeispiel ein Gebläse 162, welches ein Magnetron 164 kühlt. Die Dimensionierung des Gebläses 162 in Figur 1 ist lediglich schematisch angedeutet. Das Magnetron 164 umfasst eine Anode 166 als Senderantenne. Diese Anode 166 ragt in einen Hohlleiter 168, in welchen Mikrowellenstrahlung 172, die in dem Magnetron 164 erzeugt wird, eingekoppelt wird. Der Hohlleiter 168 ist entsprechend dimensioniert und kann beispielsweise einen quadratischen oder rechteckigen Querschnitt aufweisen. Der Hohlleiter 168 weist in dem Ausführungsbeispiel in Figur 1 einen im Wesentlichen aufwärts gerichteten, senkrechten Verlauf auf und ist an einen Gehäuseboden 170 der Durchlaufgeschirrspülmaschine 110 unterhalb der Fördereinrichtung 116 angekoppelt. Wie oben beschrieben können auch mehrere Mikrowellendesinfektionsvorrichtungen 160 mit mehreren derartiger Hohlleiter 168 vorgesehen sein, oder auch mehrere Hohlleiter 168 für ein und dieselbe Mikrowellenquelle.

Die Mikrowellendesinfektionsvorrichtung 116 bzw. das Magnetron 164 sind ausgestaltet, um Mikrowellenstrahlung 172 zu erzeugen und über den Hohlleiter 168 auf das Reinigungsgut 112 aufzubringen, wenn sich dieses im Bereich der Mündung des Hohlleiters 168 in die Desinfektionszone 153 befindet. Es können Sensoren vorgesehen sein, welche detektieren, ob sich Reinigungsgut 112 oberhalb dieser Mündung befindet, so dass vorzugsweise lediglich in diesem Fall Mikrowellenstrahlung 172 generiert wird. Alternativ oder zusätzlich lässt sich auch die Anode 166 als Detektor nutzen, um zu erkennen, ob Reinigungsgut 112 vorhanden ist, wie dies beispielsweise auch in EP 1327844 A2 vorgeschlagen wird. Auf diese Weise lässt sich Energie einsparen, indem unnötige Mikrowelleneinstrahlung vermieden wird.

Die Mikrowellenstrahlung 172 kann gemäß einem der oben beschriebenen Ausführungsbeispiele ausgestaltet sein, beispielsweise mit einer Frequenz im Bereich von 2,5 GHz, um an dem Reinigungsgut 112 anhaftendes Wasser aufzuheizen (indirekte Desinfektion), um das Reinigungsgut 112 selbst aufzuheizen (indirekte Desinfektion) und/oder um an dem Reinigungsgut 112 anhaftende Keime direkt abzutöten (direkte Desinfektion). Auch andere der oben beschriebenen Desinfektionsprinzipien sind denkbar. Auch eine Variation der eingestrahlten Frequenzbänder der Mikrowellenstrahlung 172 mit der Zeit ist denkbar. Weiterhin kann die Fördereinrichtung 116 sowie gegebenenfalls ein Korb oder eine andere Aufnahmeeinrichtung zur Aufnahme des Reinigungsguts 112 im Wesentlichen transparent für die eingestrahlte Mikrowellenstrahlung 172 ausgestaltet werden, beispielsweise durch entsprechende Wahl der Materialien.

In dem in Figur 1 dargestellten Ausführungsbeispiel ist die Mündung des Hohlleiters 168 in das Innere der Desinfektionszone 153 im Wesentlichen offen. Zusätzlich können jedoch Klappen, Ventile, Krümmungen, Mikrowellen-transparente Überdachungen oder andere Ausgestaltungen vorgesehen sein, welche zwar eine Beaufschlagung des Reinigungsgutes 112 mit Mikrowellenstrahlung 172 ermöglichen, jedoch verhindern, dass vom Reinigungsgut 112 abtropfendes Wasser bzw. andere Reinigungsflüssigkeit durch den Hohlleiter 168 zum Magnetron 164 gerät und dieses beschädigt.

Zur Kontrolle einer Temperatur des Reinigungsgutes 112 kann im Bereich der Mündung des Hohlleiters 168 in die Trocknungszone 154 ein Temperatursensor 174 vorgesehen sein. Die Signale dieses Temperatursensors 174 können beispielsweise an eine zentrale Steuerung 176 übermittelt werden, welche in Figur 1 lediglich schematisch angedeutet ist, und welche beispielsweise das Magnetron 164 ansteuern kann. Aus diesen Temperatursignalen kann die Steuerung 176 beispielsweise Wärmeäquivalente ableiten, mit denen das Reinigungsgut 112 beaufschlagt wird, und/oder kann die Desinfektion entsprechend steuern. Beispielsweise kann die Steuerung 176 die Mikrowellenfrequenz steuern, die Mikrowellenintensität, die Bandgeschwindigkeit (und damit die Einwirkzeit), eine Pulsdauer von Mikrowellenpulsen oder eine Kombination dieser Parameter. Auf diese Weise kann durch die Steuerung 176 die Desinfektionswirkung der Mikrowellendesinfektionsvorrichtung 160 gezielt gesteuert werden.

Die Steuerung 176 kann weiterhin, wie oben beschrieben, eine Schnittstelle 177 aufweisen. Beispielsweise kann diese Schnittstelle 177 genutzt werden, um einen oder mehrere Zielkeime auszuwählen oder vorzugeben, auf die die Desinfektion besonders abgestimmt werden soll. Hierzu kann auf die oben beschriebenen Möglichkeiten verwiesen werden.

Das Gebläse 162 der Mikrowellendesinfektionsvorrichtung 160 erzeugt einen in Figur 1 aufwärts gerichteten Luftstrom 178. Da das Magnetron 164 Wärme generiert, welche vom Luftstrom 178 aufgenommen wird, bewirkt dieser erwärmte Luftstrom 178 einen ersten Trocknungseffekt des Reinigungsgutes 112. Der erwärmte Luftstrom 178 steigt zur Wärmerückgewinnungseinrichtung 156 auf, wo die Wärme dieses Luftstroms 178 teilweise zurückgewonnen wird.

Als weiterer, optionaler Teil schließt sich an den Bereich 153, in welchem die Wärmerückgewinnungseinrichtung 156 und die Mikrowellendesinfektionsvorrichtung 160 aufgenommen sind, eine Trocknungszone 154 mit einer Gebläsezone 180 an. In dieser Gebläsezone 180 ist ein Trocknungsgebläse 182 aufgenommen. Aus dem Trocknungsgebläse 182 austretende erwärmte Luft wird über Austrittsdüsen 184 auf die Oberseite des zu reinigenden Gutes 112 aufgeblasen. Aufgrund der Mikrowellenstrahlung 172 und dem oben beschrieben ersten Trocknungseffekt der Mikrowellendesinfektionsvorrichtung 160 kann jedoch auf das Trocknungsgebläse 182 im Idealfall vollständig verzichtet werden, oder dieses Trocknungsgebläse 182 kann von seiner Dimensionierung her erheblich verringert werden. Auf diese Weise lässt sich ein erheblicher Energieaufwand einsparen.

Die Trocknungszone 154 ist durch einen weiteren Trennvorhang 132 gegen eine Auslaufstrecke 188 abgeschirmt. Im Bereich der Auslaufstrecke 188 der Durchlaufgeschirrspülmaschine 110 gemäß der Darstellung in Figur 1 kann das getrocknete und teilweise abgekühlte, nunmehr gereinigte Gut 112 von der als Transportband ausgebildeten Fördereinrichtung 116 entnommen werden.

Es sei darauf hingewiesen, dass auch eine andere Ausgestaltung der Zonen der Durchlaufgeschirrspülmaschine 110 als in dem in Figur 1 dargestellten Ausführungsbeispiel möglich ist, insbesondere eine andere Anordnung der Zonen oder eine andere Anzahl von Zonen. Insbesondere kann die Mikrowellendesinfektionsvorrichtung 160 an anderen Stellen angeordnet sein, beispielsweise integriert in eine andere Zone, die gleichzeitig einem anderen Zweck dient. Beispielsweise können die Trocknungszone 154 und die Desinfektionszone 153 zusammengefasst werden, oder es kann eine Mikrowellendesinfektionsvorrichtung 160 in der Spülzone 118 und/oder in der Pumpenklarspülzone 134 und/oder in der Frischwasserklarspülzone 138 vorgesehen sein. Auch am Übergang zwischen mehreren Zonen können Mikrowellendesinfektionsvorrichtungen 160 eingesetzt werden. Es können auch Kombinationen der verschiedenen, oben beschriebenen Desinfektionsprinzipien eingesetzt werden. Beispielsweise kann eine Mikrowellendesinfektionsvorrichtung 160 derart eingesetzt werden, dass durch Mikrowellenstrahlung 172 ein ohnehin in vielen Zonen vorhandener Dampf gezielt überhitzt wird, d.h. zumindest lokal auf Temperaturen über 100 °C aufgeheizt wird, um einen Desinfektionseffekt zu bewirken oder zu verstärken. Beispielsweise kann dies auch in der Desinfektionszone 153 erfolgen. Auf einen separaten Dampferzeuger, welcher in der Durchlaufgeschirrspülmaschine 110 aufgrund des hohen Luftstromes und des großen Volumens eine hohe Leistung aufweisen müsste und daher in der Praxis aus Energiegründen kaum realisierbar ist, kann dann verzichtet werden und dennoch eine Dampfdesinfektion vorgenommen werden.

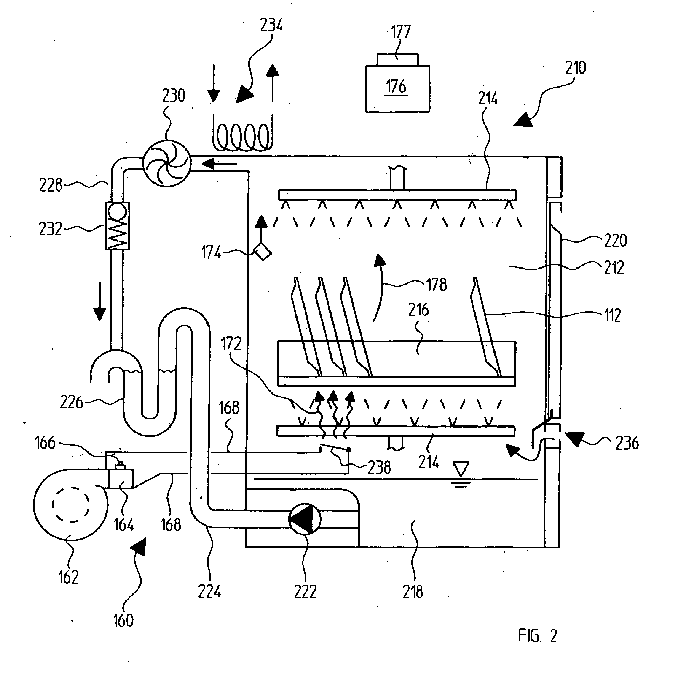

In Figur 2 ist ein zweites Ausführungsbeispiel eines Reinigungsgerätes zur Reinigung von Reinigungsgut 112 dargestellt. In diesem Fall handelt es sich bei dem Reinigungsgerät um eine Einkammergeschirrspülmaschine 210. Die Einkammergeschirrspülmaschine 210 umfasst eine Spülkammer 212 mit einem darin aufgenommenen Sprühdüsensystem 214 sowie mit entsprechenden, in Figur 2 nicht dargestellten Einrichtungen zur Beförderung von Reinigungsflüssigkeit zu diesem Sprühdüsensystem 214. Auf diese Weise kann das Reinigungsgut 112, welches in einem entsprechenden Korb 216 aufgenommen ist, aus einem Tank 218 im Bodenbereich der Spülkammer 212 mit Spülflüssigkeit beaufschlagt werden.

Die Spülkammer 212 ist durch eine Frontklappe 220 zugänglich und mit Reinigungsgut 112 beladbar. Abwasser aus dem Tank 218 kann über eine Ablaufpumpe 222 in einen Ablauf 224 abgepumpt werden. Der Ablauf 224 umfasst einen Siphonbogen 226 mit einem darin aufgenommenen Wasservorrat als Geruchssperre. Die Einkammergeschirrspülmaschine 210 weist einen Warmluftauslass 228 auf, welcher den Innenraum der Spülkammer 212 unter Umgehung des Siphonbogens 226 mit dem Ablauf 224 verbindet. In dem Warmluftauslass 228 sind in diesem Ausführungsbeispiel ein Gebläse 230 und ein Rückschlagventil 232 aufgenommen. Zudem weist der Warmluftauslass 228 eine Wärmerückgewinnungseinrichtung in Form eines Wärmetauschers 234 auf, welcher in Figur 2 lediglich symbolisch angedeutet ist. Mittels dieses Wärmetauschers 234 kann der warmen Abluft, die aus der Spülkammer 212 abgesaugt wird, Wärme zumindest teilweise entzogen werden. Die Wärmetauscherflüssigkeit kann anschließend beispielsweise dem Tank 218 zugeführt werden, so dass Energie eingespart werden kann. Unterhalb der Frontklappe 220 der Spülkammer 212 ist ein Lufteinlass 236 in Form eines Spaltes vorgesehen, durch welchen ein Druckausgleich in der Spülkammer 212 erfolgen kann. Bei diesem Lufteinlass 236 handelt es sich, wie beispielsweise auch bei dem Warmluftauslass 228, um optionale Komponenten. Die erfindungsgemäße Einkammergeschirrspülmaschine könnte auch ohne diese Komponenten realisiert werden.

Weiterhin weist die Einkammergeschirrspülmaschine 210 gemäß dem Ausführungsbeispiel in Figur 2 eine Mikrowellendesinfektionsvorrichtung 160 auf. Diese Mikrowellendesinfektionsvorrichtung 160 umfasst wiederum, wie auch in dem Ausführungsbeispiel in Figur 1, ein Gebläse 162, eine Mikrowellenquelle (hier wiederum in Form eines Magnetrons 164) mit einer Magnetron-Anode 166 und einem in das Innere der Spülkammer 212 hineinführenden Hohlleiter 168. Wiederum ist die Mikrowellendesinfektionsvorrichtung 160 in Figur 2 lediglich schematisch dargestellt und kann beispielsweise auch mehrere Mikrowellenquellen und mehrere Hohlleiter 168 umfassen.

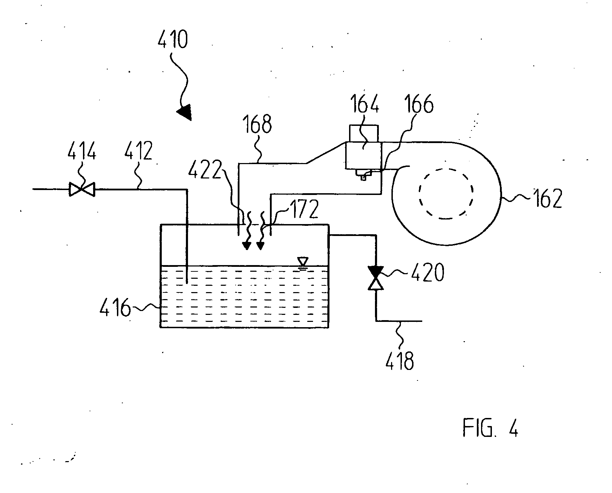

Die Mikrowellendesinfektionsvorrichtung 160 kann wiederum einen Desinfektionseffekt nach einem oder mehreren der oben beschriebenen Desinfektionsprinzipien bewirken. Insbesondere können auch wieder mehrere Mikrowellendesinfektionsvorrichtungen 160 implementiert werden, beispielsweise um das Reinigungsgut 112 von mehreren Seiten zu bestrahlen. Wiederum kann ein direkter oder indirekter Desinfektionseffekt verwendet werden, also eine direkte Keimabtötung durch die Mikrowellenstrahlung 172 und/oder eine indirekte Keimabtötung durch Erhitzung des Reinigungsgutes 112, durch Erhitzung von Reinigerflüssigkeit, durch Überhitzung von Dampf (d.h. von ohnehin in der Spülkammer 212 vorhandenem und/oder separat erzeugtem und/oder durch Mikrowellenstrahlung erzeugtem) durch Mikrowellenbestrahlung, durch Dampferzeugung mit Mikrowellenstrahlung in einem separaten Dampferzeuger (siehe auch unten Figur 4) oder durch eine beliebige Kombination dieser Prinzipien.

Bei dem in Figur 2 dargestellten Ausführungsbeispiel der Einkammergeschirrspülmaschine 210 ist die Mikrowellendesinfektionsvorrichtung 160 vorzugsweise derart ausgestattet, dass diese über ihr Gebläse 162 einen aufwärts gerichteten Luftstrom 178 generiert. Dieser Luftstrom 178 unterstützt die Trocknung des Reinigungsgutes 112 nach Durchführung einer Reinigung des Reinigungsgutes 112 mit Reinigungsflüssigkeit. Der Luftstrom kann anschließend über das Gebläse 230 in den Ablauf 224 abgesaugt werden. Aufgrund des Lufteinlasses durch das Gebläse 162 der Mikrowellendesinfektionsvorrichtung 160 kann optional auf den Lufteinlassspalt 236 verzichtet werden, wenn die beiden Gebläse 162 und 230 entsprechend aufeinander angepasst werden.

Der Hohlleiter 168 der Mikrowellendesinfektionsvorrichtung 160 weist an seinem in die Spülkammer 212 hineinragenden Ende eine aufwärts gerichtete Krümmung auf. Vorzugsweise ist der Hohlleiter 168 derart ausgestaltet, dass Reinigungsflüssigkeit nicht durch den Hohlleiter 168 zum Magnetron 164 gelangen kann. Zu diesem Zweck kann der Hohlleiter 168 beispielsweise im Bereich der Krümmung entsprechende Schlitze oder andere Öffnungen als Ablauf aufweisen. Zusätzlich oder alternativ kann der Hohlleiter 168 in diesem wie auch in den anderen Ausführungsbeispielen an seinem Ende oder in seinem Inneren ein Ventil aufweisen. Dieses Ventil ist in Figur 2 symbolisch in Form einer Klappe 238 dargestellt. Der vom Gebläse 162 generierte Luftstrom kann ausreichen, um diese Klappe 238 zu öffnen, so dass Mikrowellenstrahlung 172 ungehindert auf das Reinigungsgut 112 gelangen kann. Andere Formen der Flüssigkeitsabweisung (z.B. für Mikrowellen transparente, wasserabweisende Flächen) sind denkbar, wie oben bereits beschrieben wurde.

Wiederum weist die Einkammergeschirrspülmaschine 210 in dem in Figur 2 dargestellten Ausführungsbeispiel einen Temperatursensor 174 im Inneren der Spülkammer 212 auf, mittels dessen die beispielsweise die Mikrowellendesinfektion und/oder auch eine Trocknung, beispielsweise eine "konventionelle" Trocknung durch Heißluft und/oder eine Mikrowellentrocknung (d.h. Abdampfung des Haftwassers durch Mikrowelleneinstrahlung vom Reinigungsgut 112) überwacht werden kann. Wiederum ist ein Steuerung 176 vorgesehen, welche beispielsweise mit Signalen des Temperatursensors 174 beaufschlagt werden kann und welche den Programmablauf steuern kann. Beispielsweise kann die in Figur 2 dargestellte Einkammergeschirrspülmaschine 210 oder ein anderes Ausführungsbeispiel einer Einkammergeschirrspülmaschine ein Programm/Reinigungsverfahren durchführen, bei welchem mindestens ein Reinigungsschritt mit einer Reinigungsflüssigkeit (z.B. ein Waschprogrammschritt und/oder ein Nachspülschritt) vorgesehen ist, gefolgt von einem Desinfektionsschritt und einem optionalen Trocknungsschritt. Die Steuerung 176 kann weiterhin, wie oben beschrieben, eine Schnittstelle 177 aufweisen. Beispielsweise kann diese Schnittstelle 177 genutzt werden, um einen oder mehrere Zielkeime auszuwählen oder vorzugeben, auf die die Desinfektion besonders abgestimmt werden soll. Hierzu kann auf die oben beschriebenen Möglichkeiten verwiesen werden.

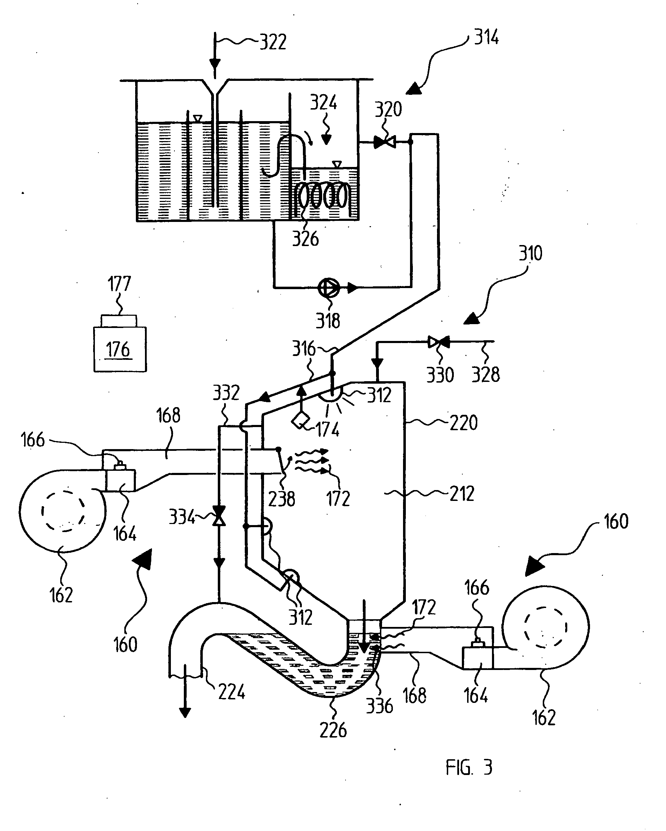

In Figur 3 ist ein drittes Ausführungsbeispiel eines Reinigungsgerätes gemäß der vorliegenden Erfindung dargestellt. In diesem Ausführungsbeispiel umfasst das Reinigungsgerät keine Geschirrspülmaschine, sondern einen Steckbeckenspüler 310. Für Details einer möglichen Ausgestaltung dieses Steckbeckenspülers 310 kann weitgehend als Beispiel auf die in DE 10348344 A1 beschriebene Ausgestaltung verwiesen werden.

Der Steckbeckenspüler 310 umfasst eine Spülkammer 212 mit einer Frontklappe 220. Mittels einer Halterung kann Reinigungsgut 112 (Halterung und Reinigungsgut sind in Figur 3 nicht dargestellt) in der Spülkammer 212 aufgenommen werden. Beispielsweise kann der Steckbeckenspüler 310 derart ausgestaltet sein, dass beim Schließen der Frontklappe 220 das Spülgut automatisch entleert wird. Auf diese Weise können flüssige oder feste Abfälle aus dem Spülgut in einen Ablauf 224 des Steckbeckenspülers 310 entleert werden. Auf diese Weise ist sichergestellt, dass der Steckbeckenspüler 310 für die Reinigung von Reinigungsgut 112 mit einem Anfall größerer Flüssigkeitsmengen geeignet ist. Dadurch unterscheidet sich der Steckbeckenspüler 310 beispielsweise von Autoklaven, welche für die Sterilisation medizinischer Instrumente eingesetzt werden. Auch in einem Autoklaven als Reinigungsgerät ist die erfindungsgemäße Mikrowellendesinfektion jedoch einsetzbar.

In der Spülkammer 212 ist das Spülgut über ein System von Düsen 312 mit Reinigungsflüssigkeit aus einer Wasser-/Dampf-Einheit 314 beaufschlagbar. Diese Wasser-/Dampf-Einheit 314 ist über ein Rohrleitungssystem 316 mit den Düsen 312 (wobei es sich bei den Düsen 312 um einfache Öffnungen und/oder auch um komplexer gestaltete Sprühdüsen handeln kann) verbunden. In dem Rohrleitungssystem 316 sind weiterhin eine Pumpe 318 sowie ein Rückschlagventil 320 aufgenommen. Die Wasser-/Dampf-Einheit 314 wird über einen Frischwasserzulauf 322 mit Frischwasser gespeist. Weiterhin umfasst die Wasser-/Dampf-Einheit 314 in diesem Ausführungsbeispiel einen optionalen "konventionellen" Dampferzeuger 324 mit einer Heizung 326, so dass das Reinigungsgut im Inneren der Spülkammer 212 über das Rohrleitungssystem 316 und die Düsen 312 mit Dampf beaufschlagt werden kann. Ein üblicher Reinigungsvorgang kann somit beispielsweise nach einem Entleeren des Reinigungsgutes 112 eine Reinigung mit Reinigungsflüssigkeit (z.B. Warm- und/oder Kaltwasser, gegebenenfalls jeweils mit Zusätzen) vorsehen, welcher ein Dampfdesinfektionsschritt folgt. Für Einzelheiten dieses Vorgangs kann beispielsweise wiederum auf die oben beschriebene DE 10348344 A1 verwiesen werden.

Alternativ oder zusätzlich kann auch ein Mikrowellen-Dampferzeuger eingesetzt werden, um Reinigungsgut 112 in der Spülkammer 212 des Steckbeckenspülers 310 zu desinfizieren. Ein Ausführungsbeispiel eines derartigen Mikrowellen-Dampferzeugers 410, welcher in dem in Figur 3 dargestellten Ausführungsbeispiel eines Reinigungsgerätes sowie auch in anderen Arten von Reinigungsgeräten eingesetzt werden kann, ist in Figur 4 dargestellt. Der Mikrowellen-Dampferzeugers 410 umfasst einen Frischwasserzufluss 412 mit einem optionalen Frischwasserventil 414. Der Frischwasserzufluss 412 ist eingerichtet, um einer Verdampfungskammer 416 Frischwasser zuzuführen. In der Verdampfungskammer 416 kann beispielsweise ein Niveausensor aufgenommen sein, um ein Überfüllen der Verdampfungskammer 416 zu verhindern.

Weiterhin weist der Mikrowellen-Dampferzeuger 410 einen Dampfauslass 418 auf, welcher in diesem Ausführungsbeispiel mit einem Rückschlagventil 420 ausgestattet ist.

Weiterhin weist der Mikrowellen-Dampferzeuger 410 eine Mikrowellenquelle auf, welche in diesem Ausführungsbeispiel wiederum als Magnetron 164 ausgebildet ist und welche ein Gebläse 162 und einen Hohlleiter 168 aufweist. Der Hohlleiter 168, welcher in diesem (nicht beschränkenden) Ausführungsbeispiel als gekrümmter Hohlleiter 168 ausgestaltet ist, mündet in die Verdampfungskammer 416. An der Mündung können wiederum Kappen, Ventile oder andere Arten von Verschlüssen vorgesehen sein, um zu verhindern, dass Feuchtigkeit (beispielsweise Wasser oder Dampf) in den Bereich des Magnetrons 164 gerät. In dem in Figur 4 dargestellten Ausführungsbeispiel ist beispielhaft eine Kappe 422 dargestellt, welche für Mikrowellen transparent ist. Weiterhin verhindert jedoch in dem in Figur 4 dargestellten Ausführungsbeispiel auch ein Luftstrom des Gebläses 162, welcher in die Verdampfungskammer 416 geleitet wird, weitgehend, dass Dampf zum Magnetron 164 gerät.

Der Luftstrom beziehungsweise Überdruck, welcher durch das Gebläse 162 erzeugt wird, kann zudem genutzt werden, um von den Mikrowellen 172 erzeugten Dampf durch den Dampfauslass 418 zu verdrängen. Dadurch wird der Überdruck in der Verdampfungskammer 416 erhöht. Diese Dampfverdrängung kann jedoch auch durch den Dampfdruck alleine erfolgen.

Der in Figur 4 dargestellte Dampferzeuger 410 kann alternativ oder zusätzlich zu dem "konventionellen" Dampferzeuger 324 der Wasser-/Dampf-Einheit 314 in Figur 3 eingesetzt werden. Beispielsweise könnte der Mikrowellen-Dampferzeuger 410 auch anstelle des Dampferzeugers 324 eingesetzt werden. Eine entsprechende Modifikation der Wasser-/Dampf-Einheit 314 ist dem Fachmann leicht möglich. Der Mikrowellen-Dampferzeuger 410 gemäß dem Ausführungsbeispiel in Figur 4 bietet gegenüber dem "konventionellen" Dampferzeuger 324 die Möglichkeit, dass auch kleine Wassermengen verdampfbar sind, wobei insbesondere in der Regel keine Mindestfüllmenge der Verdampfungskammer 416 (beispielsweise zum Bedecken einer Heizung 326) erforderlich wäre. Auf diese Weise lässt sich somit effizient Dampf erzeugen, was wiederum zu einer Energieeinsparung führen kann.

Der von dem "konventionellen" Dampferzeuger 324 und/oder dem Mikrowellen-Dampferzeuger 410 erzeugte Dampf kann in einem Dampfsterilisationsschritt in dem Steckbeckenspüler 310 gemäß Figur 3 eingesetzt werden. Zusätzlich kann dieser Dampf durch die Mikrowellendesinfektionsvorrichtung 160 gemäß Figur 3 innerhalb der Spülkammer 212 überhitzt werden, um den Dampfdesinfektionswirkungsgrad zusätzlich zu erhöhen.

Weiterhin umfasst der Steckbeckenspüler 310 gemäß dem in Figur 3 dargestellten Ausführungsbeispiel eine Zuleitung 328 mit einem Rückschlagventil 330, welche in der Spülkammer 212 mündet. Die Zuleitung 328 kann beispielsweise mit einem Frischluftgebläse (nicht dargestellt) verbunden werden und kann genutzt werden, um nach einem Dampfdesinfektionsschritt Dampf über eine Abluftleitung 332 unter Umgehung des Siphonbogens 226 in den Ablauf 224 zu verdrängen. In der Abluftleitung 232 ist wiederum ein selbst sperrendes Ventil in Form eines Rückschlagventils 334 aufgenommen. Auf diese Weise kann nach Durchführung des Dampfdesinfektionsschrittes durch Zuluft das Reinigungsgut abgekühlt und bereits teilweise getrocknet werden, und der Dampf kann zumindest teilweise aus der Spülkammer 212 in den Ablauf 224 verdrängt werden, so dass beim Öffnen der Frontklappe 220 die Arbeitsumgebung nur geringfügig belastet wird. Es sei jedoch darauf hingewiesen, dass die Zuleitung 328 und die Abluftleitung 332 jeweils optionale Komponenten sind und dass der Steckbeckenspüler 310 auch jeweils ohne diese Komponenten realisiert werden könnte oder nur mit jeweils einer dieser Komponenten.

Weiterhin umfasst der erfindungsgemäße Steckbeckenspüler 310 gemäß dem in Figur 3 dargestellten Ausführungsbeispiel wiederum eine Mikrowellendesinfektionsvorrichtung 160. Diese Mikrowellendesinfektionsvorrichtung 160 ist beispielsweise analog zu den Mikrowellendesinfektionsvorrichtungen 160 gemäß den vorgenannten Ausführungsbeispielen ausgestaltet, so dass auf die obigen Beschreibungen und Desinfektionsprinzipien verwiesen werden kann. Wiederum umfasst die Mikrowellendesinfektionsvorrichtung 160 einen Hohlleiter 168, welcher in der Spülkammer 212 mündet und über welchen das Reinigungsgut 112 mit Mikrowellenstrahlung 172 beaufschlagbar ist. Analog zum Beispiel in Figur 2 weist auch bei dem in Figur 3 dargestellten Ausführungsbeispiel vorzugsweise die Mikrowellendesinfektionsvorrichtung 160 an der Mündung des Hohlleiters 168 in die Spülkammer 212 ein selbstsperrendes Ventil auf, welches beispielsweise wiederum in Form einer Klappe 238 ausgestaltet sein kann. Diese Klappe 238 verhindert nicht nur dass Feuchtigkeit in den Hohlleiter 168 eindringen kann, sondern kann auch eine Dichtigkeit der Spülkammer 212 bei einer Verdrängung des Dampfes aus der Spülkammer 212 bewirken. So kann bei einem Einpressen von Frischluft über die Zuleitung 328 (z.B. über ein nicht dargestelltes Gebläse) die Klappe 238 durch einen leichten Überdruck in der Spülkammer 212 geschlossen sein, so dass feuchte Abluft und Dampf über die Abluftleitung 332 in den Ablauf 224 verdrängt werden können, wobei die Klappe 238 zumindest weitgehend verhindert, dass diese feuchte Luft und dieser Dampf hin zur Mikrowellenquelle bzw. dem Magnetron 164 verdrängt werden. Das Rückschlagventil 334 verhindert eine Rückverkeimung des Innenraums der Spülkammer 212 und des darin aufgenommenen Spülgutes 112.

Anschließend kann, beispielsweise im Anschluss an den beschriebenen Dampfverdrängungsschritt, die Mikrowellendesinfektionsvorrichtung 160 gestartet werden (beispielsweise wiederum über eine Steuerung 176, welche den Programmablauf entsprechend steuert), so dass das Spülgut 112 mit Mikrowellenstrahlung 172 beaufschlagt und somit direkt oder indirekt zusätzlich zu dem Dampfdesinfektionsschritt desinfiziert wird. Wiederum kann dabei durch das Gebläse 162 der Mikrowellendesinfektionsvorrichtung 160 im Inneren der Spülkammer 212 ein Luftstrom erzeugt werden, welcher die Trocknung des Reinigungsgutes 112 unterstützt. Eine Abfuhr von Überdruck, welcher durch das Gebläse 162 der Mikrowellendesinfektionsvorrichtung 160 erzeugt wird, kann über die Abluftleitung 332 in den Ablauf 224 erfolgen.

Alternativ oder zusätzlich kann die Mikrowellendesinfektionsvorrichtung 160 auch den oben beschriebenen Dampfdesinfektionsschritt unterstützen oder sogar ganz ersetzen. Bei einer Unterstützung des Dampfdesinfektionsschrittes kann beispielsweise das Prinzip der Erwärmung des Reinigungsgutes 112, eine direkte Keimabtötung durch Mikrowellenstrahlung 172, eine Dampfüberhitzung, eine Erhitzung von anhaftendem Wasser oder eine Kombination dieser und/oder anderer Prinzipien verwendet werden. Ein Programmablauf kann also ein Entleeren (optional) des Spülgutes vorsehen, eine Flüssigkeitsreinigung, einen Dampfdesinfektionsschritt (optional) und einen Mikrowellendesinfektionsschritt, sowie wiederum optional einen Trocknungs- und/oder Abkühlungsschritt, beispielsweise mit einem Dampfverdrängungsschritt. Einzelne Programmschritte sind auch wiederholt durchführbar, und die dargestellte Reihenfolge ist bevorzugt, jedoch nicht zwingend. Ein derartiger Programmablauf kann beispielsweise wiederum durch die Steuerung 176 gesteuert werden. Bei der Ausgestaltung des Reinigungsgerätes als Steckbeckenspüler 310 macht sich insbesondere die oben beschriebene Ausgestaltung positiv bemerkbar, bei welcher der Steuerung 176 über die Schnittstelle 177 von einem Benutzer (z.B. durch direkte Eingabe und/oder durch Datentransfer) ein oder mehrere Zielerreger vorgegeben werden, auf welche die Desinfektion gezielt abgestimmt werden kann.

Ein weiterer Vorteil der Mikrowellendesinfektion bei dem in Figur 3 dargestellten Ausführungsbeispiel sowie auch bei anderen Arten von Reinigungsgeräten besteht darin, dass die Mikrowellendesinfektion auch eine Trocknung des Reinigungsgutes 112 unterstützen kann. Wiederum kann auch bei dem in Figur 3 dargestellten Ausführungsbeispiel die Mikrowellendesinfektion und/oder die Trocknung, beispielsweise eine Mikrowellentrocknung oder eine "konventionelle" Trocknung, über einen Temperatursensor 174, welcher wiederum in Figur 3 lediglich symbolisch angedeutet ist, überwacht werden und beispielsweise durch die Steuerung 176 gesteuert werden.

In Figur 3 ist zudem ein weiteres Ausführungsbeispiel dargestellt, wie Mikrowellenstrahlung 172 zur Dampferzeugung und damit zur Dampfsterilisation genutzt werden kann. Diese Art der Dampferzeugung ist alternativ oder zusätzlich zu den oben beschriebenen Dampferzeugern beziehungsweise Arten der Dampferzeugung einsetzbar.

Zu diesem Zweck umfasst der Steckbeckenspüler 310 gemäß dem in Figur 3 dargestellten Ausführungsbeispiel im Bereich des Siphonbogens 226 des Ablaufs 224 eine weitere Mikrowellendesinfektionsvorrichtung 116, welche wiederum ein Magnetron 164, ein Gebläse 162 und einen Hohlleiter 168 umfasst. Durch ein Mikrowellen-transparentes Fenster 336, welches verhindert, dass Wasser aus dem Siphonbogen 226 in den Hohlleiter 168 eindringt, kann Mikrowellenstrahlung 172 genutzt werden, um Wasser im Siphonbogen 226 so zu erhitzen, dass Dampf entsteht. Dieser Dampf kann wiederum zur Sterilisationswirkung beitragen. Entsprechend kann diese Art der Dampferzeugung in die oben beschriebenen Verfahrensabläufe in einer oder mehreren Varianten eingebunden werden. Zudem kann die Erhitzung des Wasservorrates im Siphonbogen 226 auch zu einer Desinfektion dieses Wasservorrates genutzt werden, was eine Rückverkeimung des Reinigungsgutes 112 in der Spülkammer 212 verhindert und eine Geruchsbildung vermindert.

Bezugszeichenliste

| | |
|---|---|
| 110 | Durchlaufgeschirrspülmaschine |
| 112 | Reinigungsgut |
| 114 | Durchlaufrichtung |
| 116 | Fördereinrichtung |
| 118 | Spülzone |
| 120 | erstes Spülsystem |
| 122 | zweites Spülsystem |
| 123 | Reinigungsfluid |
| 124 | erste Pumpe 126 Spülzonentank |
| 128 | Pumpengehäuse |
| 130 | Tankabdecksieb |
| 132 | Trennvorhang |
| 134 | Pumpenklarspülzone |
| 136 | Trennwand |
| 138 | Frischwasserklarspülzone |
| 140 | zweite Pumpe |
| 142 | erstes Sprührohr |
| 144 | zweites Sprührohr |
| 146 | oberes Sprührohr |
| 148 | unteres Sprührohr |
| 150 | Sprührohrverlauf |
| 152 | Frischwasserstrahl |
| 153 | Desinfektionszone |
| 154 | Trocknungszone |
| 156 | Wärmerückgewinnungseinrichtung |
| 158 | Abluftgebläse |
| 160 | Mikrowellendesinfektionsvorrich- tung |
| 162 | Gebläse |
| 164 | Magnetron |
| 166 | Anode |
| 168 | Hohlleiter |
| 170 | Gehäuseboden |
| 172 | Mikrowellenstrahlung |
| 174 | Temperatursensor |
| 176 | Steuerung |
| 177 | Schnittstelle |
| 178 | Luftstrom |
| 180 | Gebläsezone |
| 182 | Trocknungsgebläse |
| 184 | Austrittsdüsen |
| 188 | Auslaufstrecke |
| 210 | Einkammergeschirrspülmaschine |
| 212 | Spülkammer |
| 214 | Sprühdüsensystem |
| 216 | Korb |
| 218 | Tank |
| 220 | Frontklappe |
| 222 | Ablaufpumpe |
| 224 | Ablauf |
| 226 | Siphonbogen |
| 228 | Warmluftauslass |
| 230 | Gebläse |
| 232 | Rückschlagventil |
| 234 | Wärmetauscher |
| 236 | Lufteinlass |
| 238 | Klappe |
| 310 | Steckbeckenspüler |
| 312 | Düsen |
| 314 | Wasser-/Dampf-Einheit |
| 316 | Rohrleitungssystem |
| 318 | Pumpe |
| 320 | Rückschlagventil |
| 322 | Frischwasserzulauf |
| 324 | Dampferzeuger |
| 326 | Heizung |
| 328 | Zuleitung |
| 330 | Rückschlagventil |
| 332 | Abluftleitung |
| 334 | Rückschlagventil |
| 336 | Mikrowellen-transparentes Fenster |
| 410 | Mikrowellen-Dampferzeuger |
| 412 | Frischwasserzufluss |
| 414 | Frischwasserventil |
| 416 | Verdampfungshammer |
| 418 | Danpfauslass |
| 420 | Rückschlagventil |
| 422 | Kappe |

**Patentansprüche**

**1.** Reinigungsgerät (110; 210; 310) zur Reinigung von Reinigungsgut (112), wobei das Reinigungsgerät (110; 210; 310) eine Reinigungszone (118, 134, 138; 212) zur Reinigung des Reinigungsgutes (112) mit einer Reinigungsflüssigkeit aufweist, wobei das Reinigungsgerät (110; 210; 310) eine Mikrowellendesinfektionsvorrichtung (160) aufweist, wobei die Mikrowellendesinfektionsvorrichtung (160) eine Mikrowellenquelle (164) zur Erzeugung von Mikrowellenstrahlung (172) aufweist, wobei die Mikrowellendesinfektionsvorrichtung (160) eingerichtet ist, um eine Hygienisierung von in dem Reinigungsgerät (110; 210; 310) aufgenommenem Reinigungsgut (112) zu bewirken, **dadurch gekennzeichnet, dass** das Reinigungsgerät (110; 210; 310) einen Steckbeckenspüler (310) zur Reinigung von medizinischem Gerät und/oder Pflegegerät aufweist, bei welchem große Mengen an zu entsorgenden Flüssigkeitsabfällen und/oder Festabfällen anfallen, wobei der Steckbeckenspüler (310) einen Ablauf (224) mit einem Siphonbogen (226) aufweist, wobei die Reinigungszone (118, 134, 138; 212) eine Spülkammer (212) umfasst, wobei der Steckbekkenspüler (310) eingerichtet ist, um Reinigungsgut (112) in der Spülkammer (212) mit der Reinigungsflüssigkeit zu beaufschlagen und wobei die Mikrowellendesinfektionsvorrichtung (160) eingerichtet ist, um das Reinigungsgut (112) in der Spülkammer (212) zumindest teilweise zu hygienisieren.

2. Reinigungsgerät (110; 210; 310) nach dem vorhergehenden Anspruch, wobei die Mikrowellendesinfektionsvorrichtung (160) eingerichtet ist, um mindestens einen der folgenden Keimreduktionsprozesse durchzuführen:

   - mittels der Mikrowellenstrahlung (172) wird in einem Dampferzeuger (410) der Mikrowellendesinfektionsvorrichtung (160) Dampf erzeugt, wobei der Dampf eingesetzt wird, um eine Keimreduktion durchzuführen;
   - die Mikrowellenstrahlung (172) wird auf das Reinigungsgut (112) ausgerichtet, wobei die Mikrowellenstrahlung (172) eingerichtet ist, um das Reinigungsgut (112) zu erwärmen;
   - die Mikrowellenstrahlung (172) wird auf das Reinigungsgut (112) ausgerichtet, wobei die Mikrowellenstrahlung (172) eingerichtet ist, um Keime auf und/oder in dem Reinigungsgut (112) und/oder in dem Reinigungsgerät (110; 210; 310) reduzieren;
   - die Mikrowellenstrahlung (172) wird auf das Reinigungsgut (112) ausgerichtet, wobei die Mikrowellenstrahlung (172) eingerichtet ist, um an dem Reinigungsgut (112) anhaftende Reinigungsflüssigkeit zu erwärmen.
   - die Mikrowellendesinfektionsvorrichtung (160) weist einen Dampferzeuger (410) auf, wobei das Reinigungsgerät (110; 210; 310) eingerichtet ist, um das Reinigungsgut (112) mit von dem Dampferzeuger (410) erzeugtem Dampf zu beaufschlagen, wobei die Mikrowellendesinfektionsvorrichtung (160) eingerichtet ist, um mittels der Mikrowellenstrahlung (172) den Dampf zu überhitzen.

3. Reinigungsgerät (110; 210; 310) nach einem der vorhergehenden Ansprüche, wobei die Mikrowellendesinfektionsvorrichtung (160) mindestens einen Hohlleiter (168) zum Ausrichten der Mikrowellenstrahlung (172) aufweist, wobei der Hohlleiter (168) im Inneren der Reinigungszone (118, 134, 138; 212) eine verschließbare Öffnung, insbesondere eine Öffnungsklappe (238), aufweist.

4. Reinigungsgerät (110; 210; 310) nach einem der vorhergehenden Ansprüche, wobei die Mikrowellendesinfektionsvorrichtung (160) eingerichtet ist, um Warmluft auf das Reinigungsgut (112) aufzubringen.

5. Reinigungsgerät (110; 210; 310) nach einem der vorhergehenden Ansprüche, wobei die Mikrowellendesinfektionsvorrichtung (160) eingerichtet ist, um das Reinigungsgut (112) mit Mikrowellen mit mindestens zwei Frequenzen zu beaufschlagen.

6. Reinigungsgerät (110; 210; 310) nach einem der vorhergehenden Ansprüche, wobei die Mikrowellendesinfektionsvorrichtung (160) eingerichtet ist, um eine oder mehrere der folgenden Mikrowellenstrahlungen (172) zu emittieren:

   - Mikrowellenstrahlung (172) mit mindestens einer Frequenz, welche zumindest teilweise vom Reinigungsgut (112) absorbiert wird,
   - Mikrowellenstrahlung (172) mit mindestens einer Frequenz, welche zumindest teilweise von an dem Reinigungsgut (112) anhaftender Reinigungsflüssigkeit absorbiert wird,
   - Mikrowellenstrahlung (172) mit mindestens einer Frequenz, welche zumindest teilweise von an dem Reinigungsgut (112) anhaftenden Keimen absorbiert wird
   - Mikrowellenstrahlung (172) mit mindestens einer Frequenz zu emittieren, welche zumindest teilweise von in dem Reinigungsgerät (110; 210; 310) enthaltenem Wasserdampf absorbiert wird.

7. Reinigungsgerät (110; 210; 310) nach einem der vorhergehenden Ansprüche, wobei die Mikrowellendesinfektionsvorrichtung (160) eingerichtet ist, um Mikrowellen in einem Frequenzband zu emittieren, wobei die Mikrowellendesinfektionsvorrichtung (160) weiterhin eingerichtet ist, um das Frequenzband zeitlich zu variieren, so dass das Frequenzband nacheinander unterschiedliche Frequenzbereiche abdeckt.

8. Reinigungsgerät (110; 210; 310) nach einem der vorhergehenden Ansprüche, wobei das Reinigungsgerät (110; 210; 310) mindestens einen Temperatursensor (174) zur Erfassung der Temperatur des Reinigungsgutes (112) aufweist.

9.  Reinigungsgerät (110; 210; 310) nach einem der vorhergehenden Ansprüche, wobei die Mikrowellendesinfektionsvorrichtung (160) mindestens eine Gebläse (162) umfasst, wobei das Gebläse (162) eingerichtet ist, um eine Trocknung des Reinigungsgutes (112) durch einen Luftstrom (178) zu unterstützen.

10. Reinigungsgerät (110; 210; 310) nach einem der vorhergehenden Ansprüche, wobei das Reinigungsgerät (110; 210; 310) eine Wärmerückgewinnungseinrichtung (156) und/oder eine Dampfniederschlagseinrichtung aufweist.

11. Reinigungsgerät (110; 210; 310) nach einem der vorhergehenden Ansprüche, wobei das Reinigungsgerät (110; 210; 310) eingerichtet ist, um einem Benutzer eine Eingabe mindestens eines Zielkeimes und/oder einer Gruppe von Zielkeimen zu ermöglichen, wobei das Reinigungsgerät (110; 210; 310) eingerichtet ist, um entsprechend dieser Auswahl die Mikrowellendesinfektionsvorrichtung (160) anzusteuern, wobei das Reinigungsgerät (110; 210; 310) eine Steuerung (176) aufweist, wobei die Steuerung (176) eingerichtet ist, um entsprechend dem ausgewählten Zielkeim und/oder der Gruppe von Zielkeimen die Mikrowellendesinfektionsvorrichtung (160) zu steuern.

12. Reinigungsgerät (110; 210; 310) nach dem vorhergehenden Anspruch, wobei das die Steuerung (176) mindestens ein Steuersignal mit mindestens einer der folgenden Informationen an die Mikrowellendesinfektionsvorrichtung (160) übermittelt:

    - eine Zielkeim-spezifische Frequenz oder ein Zielkeim-spezifisches Frequenzband;
    - eine Zielkeim-spezifische Einwirkzeit einer Desinfektion;
    - eine Zielkeim-spezifische Wirkungsdosis.

13. Reinigungsgerät (110; 210; 310) nach einem der vorhergehenden Ansprüche, wobei das Reinigungsgerät (110; 210; 310) eine Steuerung (176) zur Steuerung (176) eines Programmablaufes umfasst, wobei mindestens ein Programmschritt des Programmablaufs als Keimreduktionsschritt ausgestaltet ist.

14. Reinigungsgerät (110; 210; 310) nach einem der vorhergehenden Ansprüche, wobei der Steckbeckenspüler (310) einen Ablauf (224) aufweist, wobei die Mikrowellendesinfektionsvorrichtung (160) eingerichtet ist, um in dem Ablauf (224) aufgenommene Flüssigkeit zu erwärmen, wobei die Mikrowellendesinfektionsvorrichtung (160) eingerichtet ist, um, aus in dem Ablauf (224) aufgenommener Flüssigkeit, Dampf zu erzeugen und um mittels des Dampfes Reinigungsgut (112) in der Spülkammer (212) zumindest teilweise zu hygienisieren.

15. Verfahren zur Reinigung von Reinigungsgut (112) in einem Reinigungsgerät (110; 210; 310), wobei das Reinigungsgut (112) in mindestens einer Reinigungszone (118, 134, 138; 212) des Reinigungsgerätes (110; 210; 310) mit einer Reinigungsflüssigkeit gereinigt wird, wobei weiterhin das Reinigungsgut (112) mittels einer Mikrowellendesinfektionsvorrichtung (160) zumindest teilweise hygienisiert wird, **dadurch gekennzeichnet, dass** das Reinigungsgerät (110; 210; 310) einen Steckbekkenspüler (310) zur Reinigung von medizinischem Gerät und/oder Pflegegerät aufweist, bei welchem große Mengen an zu entsorgenden Flüssigkeitsabfällen und/oder Festabfällen anfallen, wobei der Steckbeckenspüler (310) einen Ablauf (224) mit einem Siphonbogen (226) aufweist, wobei die Reinigungszone (118, 134, 138; 212) eine Spülkammer (212) umfasst, wobei der Steckbeckenspüler (310) eingerichtet ist, um Reinigungsgut (112) in der Spülkammer (212) mit der Reinigungsflüssigkeit zu beaufschlagen und wobei die Mikrowellendesinfektionsvorrichtung (160) das Reinigungsgut (112) in der Spülkammer (212) zumindest teilweise hygienisicrt.

## Claims

1.  Cleaning appliance (110; 210; 310) for the cleaning of cleaning stock (112), the cleaning appliance (110; 210; 310) having a cleaning zone (118, 134, 138; 212) for cleaning the cleaning stock (112) with a cleaning fluid, the cleaning appliance (110; 210; 310) having a microwave disinfection apparatus (160), the microwave disinfection apparatus (160) having a microwave source (164) for the generation of microwave radiation (172), the microwave disinfection apparatus (160) being set up in order to bring about a hygienization of cleaning stock (112) received in the cleaning appliance (110; 210; 310), **characterized in that** the cleaning appliance (110; 210; 310) has a basin washer (310) for the cleaning of medical instruments and/or care instruments, in which large quantities of liquid waste and/or solid waste to be disposed of occur, the basin washer (310) having an outflow (224) with a siphon bend (226), the cleaning zone (118, 134, 138; 212) comprising a washing chamber (212), the basin washer (310) being set up in order to subject cleaning stock (112) in the washing chamber (212) to the cleaning fluid, and the microwave disinfection apparatus (160) being set up in order to at least partially hygienize the cleaning stock (112) in the washing chamber (212).

**2.** Cleaning appliance (110; 210; 310) as claimed in the preceding claim, the microwave disinfection apparatus (160) being set up in order to carry out at least one of the following germ reduction processes:

- by means of microwave radiation (172), steam is generated in a steam generator (410) of the microwave disinfection apparatus (160), the steam being used in order to carry out germ reduction;
- the microwave radiation (172) is aligned with the cleaning stock (112), the microwave radiation (172) being set up in order to heat the cleaning stock (112);
- the microwave radiation (172) is aligned with the cleaning stock (112), the microwave radiation (172) being set up in order to reduce germs on and/or in the cleaning stock (112) and/or in the cleaning appliance (110; 210; 310);
- the microwave radiation (172) is aligned with the cleaning stock (112), the microwave radiation (172) being set up in order to heat cleaning fluid adhering to the cleaning stock (112);
- the microwave disinfection apparatus (160) has a steam generator (410), the cleaning appliance (110; 210; 310) being set up in order to subject the cleaning stock (112) to steam generated by the steam generator (410), the microwave disinfection apparatus (160) being set up in order to superheat the steam by means of microwave radiation (172).

**3.** Cleaning appliance (110; 210; 310) as claimed in one of the preceding claims, the microwave disinfection apparatus (160) having at least one waveguide (168) for orienting the microwave radiation (172), the waveguide (168) having a closable orifice, in particular an opening flap (238), inside the cleaning zone (118, 134, 138; 212).

**4.** Cleaning appliance (110; 210; 310) as claimed in one of the preceding claims, the microwave disinfection apparatus (160) being set up in order to apply hot air to the cleaning stock (112).

**5.** Cleaning appliance (110; 210; 310) as claimed in one of the preceding claims, the microwave disinfection apparatus (160) being set up in order to subject the cleaning stock (112) to microwaves with at least two frequencies.

**6.** Cleaning appliance (110; 210; 310) as claimed in one of the preceding claims, the microwave disinfection apparatus (160) being set up in order to emit one or more of the following types of microwave radiation (172):

- microwave radiation (172) with at least one frequency which is absorbed at least partially by the cleaning stock (112),
- microwave radiation (172) with at least one frequency which is absorbed at least partially by the cleaning fluid adhering to the cleaning stock (112),
- microwave radiation (172) with at least one frequency which is absorbed at least partially by germs adhering to the cleaning stock (112),
- microwave radiation (172) with at least one frequency which is absorbed at least partially by steam contained in the cleaning appliance (110; 210; 310).

**7.** Cleaning appliance (110; 210; 310) as claimed in one of the preceding claims, the microwave disinfection apparatus (160) being set up in order to emit microwaves in a frequency band, the microwave disinfection apparatus (160) being set up, furthermore, in order to vary the frequency band in time, so that the frequency band successively covers different frequency ranges.

**8.** Cleaning appliance (110; 210; 310) as claimed in one of the preceding claims, the cleaning appliance (110; 210; 310) having at least one temperature sensor (174) for detecting the temperature of the cleaning stock (112).

**9.** Cleaning appliance (110; 210; 310) as claimed in one of the preceding claims, the microwave disinfection apparatus (160) comprising at least one blower (162), the blower (162) being set up in order to assist drying of the cleaning stock (112) by an air stream (178).

**10.** Cleaning appliance (110; 210; 310) as claimed in one of the preceding claims, the cleaning appliance (110; 210; 310) having a heat recovery device (156) and/or a steam precipitation device.

**11.** Cleaning appliance (110; 210; 310) as claimed in one of the preceding claims, the cleaning appliance (110; 210; 310) being set up in order to enable a user to input at least one target germ and/or one group of target germs, the cleaning appliance (110; 210; 310) being set up in order to activate the microwave disinfection apparatus (160) according to this selection, the cleaning appliance (110; 210; 310) having a control (176), the control (176) being set up in order to control the microwave disinfection apparatus (160) according to the selected target germ and/or the group of target germs.

**12.** Cleaning appliance (110; 210; 310) as claimed in the preceding claim, the control (176) transmitting at least one control signal with at least one of the following information items to the microwave disinfection apparatus (160):

- a target germ-specific frequency or a target germ-specific frequency band;
- a target germ-specific action time of a disinfection;
- a target germ-specific active dose.

13. Cleaning appliance (110; 210; 310) as claimed in one of the preceding claims, the cleaning appliance (110; 210; 310) comprising a control (176) for the control (176) of a program sequence, at least one program step of the program sequence being configured as a germ reduction step.

14. Cleaning appliance (110; 210; 310) as claimed in one of the preceding claims, the basin washer (310) having an outflow (224), the microwave disinfection apparatus (160) being set up in order to heat fluid received in the outflow (224), the microwave disinfection apparatus (160) being set up in order to generate steam from fluid received in the outflow (224) and in order to at least partially hygienize cleaning stock (112) in the washing chamber (212) by means of the steam.

15. Method for the cleaning of cleaning stock (112) in a cleaning appliance (110; 210; 310), the cleaning stock (112) being cleaned with a cleaning fluid in at least one cleaning zone (118, 134, 138; 212) of the cleaning appliance (110; 210; 310), the cleaning stock (112) being, furthermore, at least partially hygienized by means of a microwave disinfection apparatus (160), **characterized in that** the cleaning appliance (110; 210; 310) has a basin washer (310) for the cleaning of medical instruments and/or care instruments, in which large quantities of liquid waste and/or solid waste to be disposed of occur, the basin washer (310) having an outflow (224) with a siphon bend (226), the cleaning zone (118, 134, 138; 212) comprising a washing chamber (212), the basin washer (310) being set up in order to subject cleaning stock (112) in the washing chamber (212) to the cleaning fluid, and the microwave disinfection apparatus (160) at least partially hygienizing the cleaning stock (112) in the washing chamber (212).

## Revendications

1. Appareil de nettoyage (110 ; 210 ; 310) pour le nettoyage d'un article à nettoyer (112), l'appareil de nettoyage (110 ; 210 ; 310) présentant une zone de nettoyage (118, 134, 138 ; 212) pour le nettoyage de l'article à nettoyer (112) avec un liquide de nettoyage, l'appareil de nettoyage (110 ; 210 ; 310) présentant un dispositif de désinfection par micro-ondes (160), le dispositif de désinfection par micro-ondes (160) présentant une source de micro-ondes (164) pour générer un rayonnement micro-ondes (172), le dispositif de désinfection par micro-ondes (160) étant conçu pour obtenir une hygiénisation de l'article à nettoyer (112) placé dans l'appareil de nettoyage (110 ; 210 ; 310), **caractérisé en ce que** l'appareil de nettoyage (110 ; 210 ; 310) présente un dispositif de lavage pour bassin hygiénique (310) pour le nettoyage d'un appareil médical et/ou d'un appareil de soins, dans lequel on obtient de grandes quantités de résidus solides et/ou de résidus liquides à éliminer, le dispositif de lavage pour bassin hygiénique (310) présentant une sortie (224) comprenant un coude de siphon (226), la zone de nettoyage (118, 134, 138 ; 212) comprenant une chambre de lavage (212), le dispositif de lavage pour bassin hygiénique (310) étant conçu pour solliciter l'article à nettoyer (112) dans la chambre de lavage (212) avec le liquide de nettoyage et dans lequel le dispositif de désinfection par micro-ondes (160) est conçu pour l'hygiénisation au moins partielle de l'article à nettoyer (112) dans la chambre de lavage (212).

2. Appareil de nettoyage (110; 210; 310) selon la revendication précédente, dans lequel le dispositif de désinfection par micro-ondes (160) est conçu pour mettre en oeuvre au moins un des processus de réduction de germes indiqués ci-après :

- au moyen du rayonnement micro-ondes (172), de la vapeur est générée dans un générateur de vapeur (410), la vapeur étant mise en oeuvre pour procéder à une réduction des germes ;
- le rayonnement micro-ondes (172) est orienté vers l'article à nettoyer (112), le rayonnement micro-ondes (172) étant conçu pour réchauffer l'article à nettoyer (112) ;
- le rayonnement micro-ondes (172) est orienté vers l'article à nettoyer (112), le rayonnement micro-ondes (172) étant conçu pour réduire les germes sur et/ou dans l'article à nettoyer (112) et/ou dans l'appareil de nettoyage (110 ; 210 ; 310) ;
- le rayonnement micro-ondes (172) est orienté vers l'article à nettoyer (112), le rayonnement micro-ondes (172) étant conçu pour chauffer le liquide de nettoyage qui adhère à l'article à nettoyer (112) ;
- le dispositif de désinfection par micro-ondes (160) présente un générateur de vapeur (410), l'appareil de nettoyage (110 ; 210 ; 310) étant conçu pour solliciter l'article à nettoyer (112) avec la vapeur générée par le générateur de vapeur (410), le dispositif de désinfection par micro-ondes (160) étant conçu pour surchauffer la vapeur au moyen du rayonnement micro-ondes (172).

**3.** Appareil de nettoyage (110 ; 210 ; 310) selon l'une quelconque des revendications précédentes, dans lequel le dispositif de désinfection par micro-ondes (160) présente au moins un guide d'ondes (168) pour l'orientation du rayonnement micro-ondes (172), le guide d'ondes (168) présentant, à l'intérieur de la zone de nettoyage (118, 134, 138 ; 212), une ouverture qui peut être fermée, en particulier un clapet d'ouverture (238).

**4.** Appareil de nettoyage (110 ; 210 ; 310) selon l'une quelconque des revendications précédentes, dans lequel le dispositif de désinfection par micro-ondes (160) est conçu pour appliquer de l'air chaud sur l'article à nettoyer (112).

**5.** Appareil de nettoyage (110 ; 210 ; 310) selon l'une quelconque des revendications précédentes, dans lequel le dispositif de désinfection par micro-ondes (160) est conçu pour solliciter l'article à nettoyer (112) avec des micro-ondes comprenant au moins deux fréquences.

**6.** Appareil de nettoyage (110 ; 210 ; 310) selon l'une quelconque des revendications précédentes, dans lequel le dispositif de désinfection par micro-ondes (160) est conçu pour émettre un ou plusieurs des rayonnements micro-ondes (172) indiqués ci-après :

    - un rayonnement micro-ondes (172) comprenant au moins une fréquence, qui est absorbé au moins en partie par l'article à nettoyer (112) ;
    - un rayonnement micro-ondes (172) comprenant au moins une fréquence, qui est absorbé au moins en partie par le liquide de nettoyage qui adhère à l'article à nettoyer (112) ;
    - un rayonnement micro-ondes (172) comprenant au moins une fréquence, qui est absorbé au moins en partie par les germes qui adhèrent à l'article à nettoyer (112) ;
    - un rayonnement micro-ondes (172) comprenant au moins une fréquence, qui est absorbé au moins en partie par la vapeur d'eau que contient l'appareil de nettoyage (110 ; 210 ; 310).

**7.** Appareil de nettoyage (110 ; 210 ; 310) selon l'une quelconque des revendications précédentes, dans lequel le dispositif de désinfection par micro-ondes (160) est conçu pour émettre des micro-ondes dans une bande de fréquences, le dispositif de désinfection par micro-ondes (160) étant en outre conçu pour soumettre la bande de fréquences à des variations temporelles, si bien que la bande de fréquences recouvre des plages de fréquences successivement différentes.

**8.** Appareil de nettoyage (110 ; 210 ; 310) selon l'une quelconque des revendications précédentes, dans lequel l'appareil de nettoyage (110 ; 210 ; 310) présente au moins un capteur de température (174) pour enregistrer la température de l'article à nettoyer (112).

**9.** Appareil de nettoyage (110 ; 210 ; 310) selon l'une quelconque des revendications précédentes, dans lequel le dispositif de désinfection par micro-ondes (160) comprend au moins un ventilateur (162), le ventilateur (162) étant conçu pour soutenir un séchage de l'article à nettoyer (112) via un courant d'air (178).

**10.** Appareil de nettoyage (110 ; 210 ; 310) selon l'une quelconque des revendications précédentes, dans lequel l'appareil de nettoyage (110 ; 210 ; 310) présente un dispositif de récupération de chaleur (156) et/ou un dispositif de précipitation de vapeur.

**11.** Appareil de nettoyage (110 ; 210 ; 310) selon l'une quelconque des revendications précédentes, dans lequel l'appareil de nettoyage (110 ; 210 ; 310) est conçu pour permettre à un utilisateur d'alimenter au moins un germe cible et/ou un groupe de germes cibles, dans lequel l'appareil de nettoyage (110 ; 210 ; 310) est conçu pour exciter le dispositif de désinfection par micro-ondes (160) conformément à cette sélection, l'appareil de nettoyage (110; 210; 310) présentant une commande (176), la commande (176) étant conçue pour commander le dispositif de désinfection par micro-ondes (160) de manière correspondante au germe cible sélectionné et/ou au groupe de germes cibles.

**12.** Appareil de nettoyage (110 ; 210 ; 310) selon l'une quelconque des revendications précédentes, dans lequel la commande (176) transmet au dispositif de désinfection par micro-ondes (160) au moins un signal de commande comprenant au moins une des informations suivantes :

    - une fréquence spécifique à un germe cible ou bien une bande de fréquences spécifique à un germe cible ;
    - une durée d'action d'une désinfection spécifique à un germe cible ;
    - une dose d'efficacité spécifique à un germe cible.

**13.** Appareil de nettoyage (110 ; 210 ; 310) selon l'une quelconque des revendications précédentes, dans lequel l'appareil de nettoyage (110 ; 210 ; 310) présente une commande (176) pour la commande (176) d'un déroulement de programme, au moins une étape du programme dans le déroulement du programme étant réalisée sous la forme d'une étape de réduction des germes.

**14.** Appareil de nettoyage (110 ; 210 ; 310) selon l'une quelconque des revendications précédentes, dans lequel le dispositif de lavage pour bassin hygiénique (310) présente une sortie (224), le dispositif de désinfection par micro-ondes (160) étant conçu pour réchauffer le liquide qui se retrouve dans la sortie (224), le dispositif de désinfection par micro-ondes (160) étant conçu pour générer de la vapeur à partir du liquide qui se retrouve dans la sortie (224) et pour soumettre l'article à nettoyer (112) à une hygiénisation au moins partielle au moyen de la vapeur, dans la chambre de lavage (212).

**15.** Procédé pour le nettoyage d'un article à nettoyer (112) dans un appareil de nettoyage (110 ; 210 ; 310), l'article à nettoyer (112) étant lavé dans au moins une zone de nettoyage (118, 134, 138 ; 212) de l'appareil de nettoyage (110 ; 210 ; 310) avec un liquide de nettoyage, dans lequel, en outre, l'article à nettoyer (112) est soumis à une hygiénisation au moins partielle au moyen d'un dispositif de désinfection par micro-ondes (160), **caractérisé en ce que** l'appareil de nettoyage (110 ; 210 ; 310) présente un dispositif de lavage pour bassin hygiénique (310) pour le nettoyage d'un appareil médical et/ou d'un appareil de soins, dans lequel on obtient de grandes quantités de résidus solides et/ou de résidus liquides à éliminer, le dispositif de lavage pour bassin hygiénique (310) présentant une sortie (224) comprenant un coude de siphon (226), la zone de nettoyage (118, 134, 138 ; 212) comprenant une chambre de lavage (212), le dispositif de lavage pour bassin hygiénique (310) étant conçu pour solliciter l'article à nettoyer (112) dans la chambre de lavage (212) avec le liquide de nettoyage et dans lequel le dispositif de désinfection par micro-ondes (160) est conçu pour l'hygiénisation au moins partielle de l'article à nettoyer (112) dans la chambre de lavage (212).

FIG 1

FIG. 2

FIG. 3

FIG. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1327844 A2
- DE 10348344 A1